# EUROPEAN PATENT SPECIFICATION

(11) **EP 1 868 994 B1**
(45) Date of publication and mention of the grant of the patent: **25.05.2011**
(21) Application number: 06724113.3
(22) Date of filing: 07.04.2006
(51) Int. Cl.: C07D 207/48, C07D 401/12, A61K 31/40, A61K 31/4025, A61P 35/00, A61P 19/02, A61P 25/00, C07D 409/12, C07D 417/12, C07D 403/12

(54) **SULFONYLPYRROLES AS HISTONE DEACETYLASE INHIBITORS**
SULFONYLPYRROLE ALS HISTONDEACETYLASEINHIBITOREN
SULFONYLPYRROLES EN TANT QU'INHIBITEURS D'HISTONE DEACETYLASE

(30) Priority: 07.04.2005 EP 05102750
(43) Date of publication of application: 26.12.2007
(73) Proprietor: 4SC AG, 82152 Planegg - Martinsried (DE)
(72) Inventor: MAIER, Thomas, 78333 Stockach (DE); BÄR, Thomas, 78479 Reichenau (DE); BECKERS, Thomas, 78464 Konstanz (DE); ZIMMERMANN, Astrid, 78467 Konstanz (DE); DULLWEBER, Frank, 78464 Konstanz (DE); GEKELER, Volker, 78465 Konstanz (DE)
(74) Representative: Kilger, Ute
(86) International application number: PCT/EP2006/003171
(87) International publication number: WO 2006/105979

(56) References cited:
- EP-A- 0 570 594
- WO-A-01/38322
- RINO RAGNO ET AL: "3-(4-Aroyl-1-methyl-1H-pyrrol-2-yl)-N-hyd roxy-2-propenamides as a new class of synthetic histone deacetylase inhibitors. 3. Discovery of novel lead compounds through structure-based drug design and docking studies" JOURNAL OF MEDICINAL CHEMISTRY, vol. 47, no. 6, 2004, pages 1351-1359, XP002339636 cited in the application

## Description

### Field of application of the invention

The Invention relates to novel sulphonylpyrrole derivatives, which are used in the pharmaceutical Industry for the production of pharmaceutical compositions.

### Known technical background

Transcriptional regulation in cells is a complex biological process. One basic principle is regulation by posttranslational modification of histone proteins, namely histone proteins H2A/B, H3 and H4 forming the octameric histone core complex. These complex N-terminal modifications at lysine residues by acetylation or methylation and at serine residues by phosphorylation constitute part of the so called "histone code" (Strahl & Ellis, Nature 403, 41-45, 2000). In a simple model, acetylation of positively charged lysine residues decreases affinity to negatively charged DNA, which now becomes accessible for the entry of transcription factors.

Histone acetylation and deacetylation is catalysed by histone acetyltransferases (HATs) and histone deacetylases (HDACs). HDACs are associated with transcriptional repressor complexes, switching chromatin to a transcriptionalty inactive, silent structure (Marks et al. Nature Cancer Rev 1, 194-202, 2001). The opposite holds true for HATs which are associated with transcriptional activator complexes. Three different classes of HDACs have been described so far, namely class I (HDAC 1-3, 8) with Mr = 42-55 kDa primarily located in the nucleus and sensitive towards inhibition by Trichostatin A (TSA), class II (HDAC 4-7, 9, 10) with Mr = 120-130kDa and TSA sensitivity and class III (Sir2 homologues) which are quite distinct by their NAD⁺ dependency and TSA Insensitivity (Ruijter et al: Biochem.J. 370, 737-749, 2003; Khochbin et al. Curr Opin Gen Dev 11, 162-166, 2001; Verdin et al. Trends Gen 19, 286-293, 2003). HDAC 11 with Mr = 39kDa was cloned recently and displayed homology to class I and II family members (Gao et al. J Biol Chem 277,25748-25755, 2002). HATs and HDACs exist in large complexes together with transcription factor and platform proteins in cells (Fischle et al. Mol Cell 9, 45-47, 2002). Surprisingly, only about 2% of all genes are regulated by histone acetylation as estimated based on differential display analysis of 340 genes and TSA as the reference HDI (von Lint et al. Gene Expression 5, 245-253, 1996). New studies with SAHA in multiple myeloma cells showed that these transcriptional changes can be grouped into distinct functional gene classes important for e.g. regulation of apoptosis or proliferation (Mitsiades et al. Proc Natl Acad Sci 101, pp540, 2004).

Substrates different to histone proteins exist For HDACs these include transcription factors like p53 and TFII E / or chaperones like Hsp90 (Johnstone & Licht, Cancer Cell 4, 13-18, 2003). Therefore the correct name for HDACs would be lysine-specific protein deacetylases. As a consequence of these findings, inhibitors of HDACs effect not only chromatin structure and gene transcription but also protein function and stability by regulating protein acetylation in general. This function of HDACs in protein acetylation might also be important for understanding of immediate gene repression by treatment with HDIs (von Lint et al. Gene Expression 5, 245-253, 1996). In this regard, proteins involved in oncogenic transformation, apoptosis regulation and malignant cell growth are of particular importance.

Different publications highlight the importance of histone acetylation for cancer development (reviewed by Kramer et al. Trends Endocrin Metabol 12, 294-300, 2001; Marks et al. Nature Cancer Rev 1, 194-202, 2001). These diseases include
(i) mutations of the HAT cAMP response element binding protein (CBP) associated with Rubinstein-Taybi syndrome, a cancer predisposition (Murata et al. Hum Mol Genet 10, 1071-1076, 2001),
(ii) aberrant recruitment of HDAC1 activity by transcription factors in acute promyelocytic leukemia (APL) by the PML-retinoic acid receptor α fusion gene (He et al. Nat genet 18, 126-135, 1998)
(iii) aberrant recruitment of HDAC activity by the overexpressed BCL6 protein in non-Hodgkins lymphoma (Dhordain et al. Nuceic Acid Res 26, 4645-4651, 1998) and finally
(iv) aberrant recruitment of HDAC activity by the AML-ETO fusion protein in acute myelogenous leukemia (AML M2 subtype; Wang et al. Proc Natl Acad Sci USA 95, 10860-10865, 1998). In this AML subtype, the recruitment of HDAC1 activity causally leads to gene silencing, a differentiation block and oncogenic transformation.
(v) HDAC1 gene knock-out in mice showed that HDAC1 has a profound function in embryonal stem cell proliferation by repressing cyclin-dependent kinase inhibitors p21^{waf1} and p27^{Klp1} (Lagger et al. Embo J. 21, 2672-2681, 2002). Since p21^{waf1} is induced by HDIs in many cancer cell lines, HDAC1 might be a crucial component in cancer cell proliferation as well. Initial siRNA based gene-knock down experiments in HeLa cells support this hypothesis (Glaser et al. 310, 529-536, 2003).
(vi) HDAC2 is overexpressed in colon carcinoma upon constitutive activation of the wnt /ß-catenin/TCF signalling pathay by loss of functional adenomatosis polyposis coli (APC) protein as reported by Zhu et al. recently (Cancer cell 5, 455-463, 2004).

On the molecular level, a pleithora of published data with various HDAC inhibitors like Trichostatin A (TSA) showed that many cancer relevant genes are up- or down regulated. These include p21^{waf1}, Cyclin E, transforming growth factor ß (TGFß), p53 or the von Hippel-Lindau (VHL) tumor suppressor genes, which are upregulated, whereas Bcl-XL, bcl2, hypoxia inducible factor (HIF)1α, vascular endothelial growth factor (VEGF) and cyclin A/D are down-regulated by HDAC inhibition (reviewed by Kramer et al. Trends Endocrin Metabol 12, 294-300, 2001). HDAC inhibitors arrest cells at G1 and G2/M within the cell cycle and deplete S-phase cells, as shown for Depsipeptide as an example (Sandor et al., British J Cancer 83, 817-825, 2000). HDAC inhibitory compounds induce p53 and caspase3/8 independent apoptosis and have broad anti-tumor activity. Anti-angiogenic activity was described also, which might be related to down-regulation of VEGF and HIF1α. In summary, HDAC inhibition effects tumor cells at different molecular levels and multiple cellular proteins are targeted. Interestingly, HDAC inhibitors were found to induce cellular differentiation and this pharmacological activity might contribute to their anti-cancer activity as well. For example it was shown recently that suberoylanilide hydroxamic acid (SAHA) induces differentiation of breast cancer cell lines, exemplified by resynthesis of milk fat membrane globule protein (MFMG), milk fat globule protein and lipid (Munster et al. Cancer Res. 61, 8492, 2001).

There is growing rational for synergism of HDAC inhibitors with chemotherapeutic as well as target specific cancer drugs. For example, synergism was shown for SAHA with the kinase / cdk inhibitor flavopiridol (Alemenara et al. Leukemia 16, 1331-1343, 2002), for LAQ-824 with the bcr-abl kinase inhibitor Glivec in CML cells (Nimmanapalli et al. Cancer Res. 63, 5126-5135, 2003), for SAHA and Trichostatin A (TSA) with etoposide (VP16), cisplatin and doxorubicin (Kim et al. Cancer Res. 63, 7291-7300, 2003) and LBH589 with the hsp90 inhibitor 17-allyl-amino-demethoxy-geldanamycin (17-AAG; George et al. Blood online, Oct.28, 2004). Also it was shown that HDAC inhibition causes reexpression of estrogen or androgen receptors in breast and prostate cancer cells with the potential to resensitize these tumors to anti-hormone therapy (Yang et al. Cancer Res. 60, 6890-6894, 2000; Nakayama et al. Lab Invest 80, 1789-1796, 2000).

HDAC inhibitors from various chemical classes were described in the literature with four most important classes, namely (i) hydroxamic acid analogs, (ii) benzamide analogs, (iii) cyclic peptides / peptolides and (iv) fatty acid analogs. A comprehensive summary of known HDAC inhibitors was published recently by Miller et al. (J Med Chem 46, 5097-5116, 2003). There is only limited data published regarding specificity of these histone deacetylase inhibitiors. In general most hydroxamate based HDI are not specific regarding class I and II HDAC enzymes. For example. TSA inhibits HDACs 1, 3, 4, 6 and 10 with IC₅₀ values around 20nM, whereas HDAC8 was inhibited with IC₅₀ = 0.49 µM (Tatamiya et al, AACR Annual Meeting 2004, Abstract #2451). But there are exceptions like the experimental HDI Tubacin, selective for the class II enzyme HDAC 6 (Haggarty et al. Proc natl Acad Sci USA 100, 4389-4394, 2003). In addition, data on class I selectivity of benzamid HDIs are emerging. MS-275 inhibited class I HDAC1 and 3 with IC₅₀ = 0.51 µM and 1.7µM, respectively. In contrast class II HDACs 4, 6, 8 and 10 were inhibited with IC₅₀ values of >100µM, >100µM, 82.5µM and 94.7µM, respectively (Tatamiya et al, AACR Annual Meeting 2004, Abstract #2451). So far it is not clear if specificity towards HDAC class I or II enzymes or a defined single isoenzyme should be superior regarding therapeutic efficacy and index.

Clinical studies in cancer with HDAC inhibitors are on-going, namely with SAHA (Merck Inc.), Valproic acid, FK228 / Depsipeptide (Gloucester Pharmaceuticals / NCI), MS275 (Berlex-Schering ), NVP LBH-589 (Novartis), PXD-101 (Topotarget / Curagen), MGCD0103 (Methylgene Inc)and Pivaloyloxymethylbutyrate / Pivanex (Titan Pharmaceuticals). These studies showed first evidence of clinical efficacy, highlighted recently by partial and complete responses with FK228 / Depsipeptide in patients with peripheral T-cell lymphoma (Plekarz et al. Blood, 98, 2865-2868, 2001) and diffuse large B-cell lymphoma by SAHA (Kelly et al. J. Clin. Oncol. 23, 3923-3931, 2005).

Recent publications also showed possible medical use of HDAC inhibitors in disease different to cancer. These diseases include systemic lupus erythematosus (Mishra et a. J Clin Invest 111, 539-552, 2003, Reilly et al. J. Immunol. 173, 4171-4178, 2004), rheumatoid arthritis (Chung et al. Mol Therapy 8, 707-717, 2003; Nishida et al. Arthritis & Rheumatology 50, 3365-3376, 2004), inflammatory diseases (Leoni et al. Proc Natl Acad Sci USA 99, 2995-3000, 2002) and neurodegenerative diseases like Huntington's disease (Steffan et al. Nature 413, 739-743, 2001, Hockly et al. Proc Natl Acad Sci USA 100(4):2041-6, 2003).

Cancer chemotherapy was established based on the concept that cancer cells with uncontrolled proliferation and a high proportion of cells in mitosis are killed preferentially. Standard cancer chemotherapeutic drugs finally kill cancer cells upon induction of programmed cell death ("apoptosis") by targeting basic cellular processes and molecules, namely RNA/DNA (alkylating and carbamylating agents, platin analogs and topoisomerase inhibitors), metabolism (drugs of this class are named antimetabolites) as well as the mitotic spindle apparatus (stabilizing and destabilizing tubulin inhibitors). Inhibitors of histone deacetylases (HDIs) constitute a new class of anti cancer drugs with differentiation and apoptosis inducing activity. By targeting histone deacetylases, HDIs effect histone (protein) acetylation and chromatin structure, inducing a complex transcriptional reprogramming, exemplified by reactivation of tumor suppressor genes and repression of oncogenes. Beside effecting acetylation of N-terminal lysine residues in core histone proteins, non-histone targets important for cancer cell biology like heat-shock-protein 90 (Hsp90) or the p53 tumor suppressor protein exist. The medical use of HDIs might not be restricted to cancer therapy, since efficacy in models for inflammatory diseases, rheumatoid arthritis and neurodegeneration was shown.

Benzoyl or acetyl substituted pyrrolyl propenamides are described in the public literature as HDAC-inhibitors, whereas the connectivity of the acyl-group is at position 2 or 3 of the pyrrole scaffold. (Mai et.al., Journal Med.Chem. 2004, Vol. 47, No. 5, 1098-1109; or Ragno et al., Journal Med.Chem. 2004, Vol. 47, No. 5, 1351-1359). Further pyrrolyl substituted hydroxamic acid derivatives are described in US4960787 as lipoxygenase inhibitors or in US6432999 as cyclooxygenase inhibitors or in EP570594 as inhibitors of cell growth.

Various compounds, which are said to be HDAC inhibitors, are reported in WO 01/38322; Journal Med. Chem. 2003, Vol. 46, No. 24, 5097-5116; Journal Med. Chem. 2003, Vol. 46, No. 4, 512-524; Journal Med. Chem. 2003, Vol. 46, No. 5, 820-830; and in Current Opinion Drug Discovery 2002, Vol. 5, 487-499.

There remains a need in the art for new, well-tolerated and more efficacious inhibitors of HDACs.

### Description of the invention

It has now been found that the N-sulphonylpyrrole derivatives, which are described in greater details below, differ profoundly from prior art compounds and are effective inhibitors of histone deacetylases and have surprising and particularly advantageous properties.

The invention thus relates to compounds of formula I 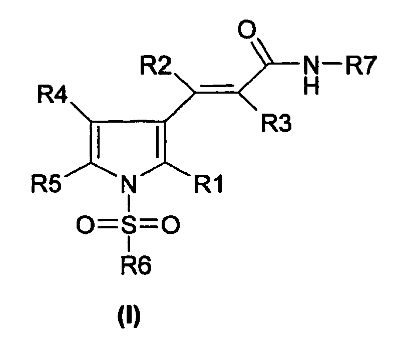 in which
- R1: is hydrogen, 1-4C-alkyl, halogen, or 1-4C-alkoxy,
- R2: is hydrogen or 1-4C-alkyl,
- R3: is hydrogen or 1-4C-alkyl,
- R4: is hydrogen, 1-4C-alkyl, halogen, or 1-4C-alkoxy,
- R5: is hydrogen, 1-4C-alkyl, halogen, or 1-4C-alkoxy,

- R6: is-T1-Q1, in which
- T1: is a bond, or 1-4C-alkylene,
- Q1: is naphthyl, HAR, or R61- and/or R62-substituted AR, in which
- AR: is naphthyl, or HAR, in which
- HAR: is a monocyclic or fused bicyclic 5- to 10-membered unsaturated heteroaromatic ring comprising one to three heteroatoms, each of which is selected from the group consisting of nitrogen, oxygen and sulfur,

- R61: is 1-4C-alkyl, or-T2-N(R611)R612, in which
- either:
- T2: is a bond, and
- R611: is hydrogen, 1-4C-alkyl, hydroxy-2-4C-alkyl, 1-4C-alkoxy-2-4C-alkyl, phenyl-1-4C-alkyl, or Har1-1-4C-alkyl, in which
- Har1: is optionally substituted by R6111 and/or R6112, and is a monocyclic or fused bicyclic 5- to 10-membered unsaturated heteroaromatic ring comprising one to three heteroatoms, each of which is selected from the group consisting of nitrogen, oxygen and sulfur, in which
- R6111: is halogen, or 1-4C-alkyl,
- R6112: is 1-4C-alkyl, and
- R612: is hydrogen, 1-4C-alkyl, 1-4C-alkoxy-2-4C-alkyl or hydroxy-2-4C-alkyl,
- or R611: and R612 together and with inclusion of the nitrogen atom, to which they are bonded, form a heterocyclic ring Het1, in which
- Het1: is morpholino, thiomorpholino, S-oxo-thiomorpholino, S,S-dioxo-thiomorpholino, piperidino, pyrrolidino, piperazino, or 4N-(1-4C-alkyl)-piperazino,
- or:
- T2: is 1-4C-alkylene, or 2-4C-alkylene interrupted by oxygen, and
- R611: is hydrogen, 1-4C-alkyl, hydroxy-2-4C-alkyl, 1-4C-alkoxy-2-4C-alkyl, phenyl-1-4C-alkyl, or Har1-1-4C-alkyl, in which
- Har1: is optionally substituted by R6111 and/or R6112, and is a monocyclic or fused bicyclic 5- to 10-membered unsaturated heteroaromatic ring comprising one to three heteroatoms, each of which is selected from the group consisting of nitrogen, oxygen and sulfur, in which
- R6111: is halogen, or 1-4C-alkyl,
- R6112: is 1-4C-alkyl, and
- R612: is hydrogen, 1-4C-alkyl, 1-4C-alkoxy-2-4C-alkyl or hydroxy-2-4C-alkyl,
- or R611 and R612: together and with inclusion of the nitrogen atom, to which they are bonded, form a heterocyclic ring Het1, in which
- Het1: is morpholino, thiomorpholino, S-oxo-thiomorpholino, S,S-dioxo-thiomorpholino, piperidino, pyrrolidino, piperazino, 4N-(1-4C-alkyl)-piperazino, imidazolo, pyrrolo, triazolo or pyrazolo,
- R62: is 1-4C-alkyl, 1-4C-alkoxy, halogen, cyano, 1-4C-alkoxy-1-4C-alkyl, 1-4C-alkylcarbonylamino or 1-4C-alkylsulphonylamino,
- R7: is hydroxyl, or Cyc1, in which
- Cyc1: is a ring system of formula la 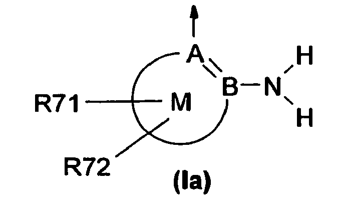
in which
- A: is C (carbon),
- B: is C (carbon),
- R71: is hydrogen, halogen, 1-4C-alkyl, or 1-4C-alkoxy,
- R72: is hydrogen, halogen, 1-4C-alkyl, or 1-4C-alkoxy,
- M: with inclusion of A and B is either a ring Ar2 or a ring Har2, in which
- Ar2: is a benzene ring,
- Har2: is a monocyclic 5- or 6-membered unsaturated heteroaromatic ring comprising one to three heteroatoms, each of which is selected from the group consisting of nitrogen, oxygen and sulfur,
and the salts of these compounds.

1-4C-Alkyl represents a straight-chain or branched alkyl radical having 1 to 4 carbon atoms. Examples which may be mentioned are the butyl, isobutyl, sec-butyl, tert-butyl, propyl, isopropyl and preferably the ethyl and methyl radicals.

2-4C-Alkyl represents a straight-chain or branched alkyl radical having 2 to 4 carbon atoms. Example which may be mentioned are the butyl, isobutyl, sec-butyl, tert-butyl, isopropyl and preferably the ethyl and propyl radicals.

1-4C-Alkylene is a branched or, particularly, straight chain alkylene radical having 1 to 4 carbon atoms. Examples which may be mentioned are the methylene (-CH₂-), ethylene (-CH₂-CH₂-), trimethylene (-CH₂-CH₂-CH₂-) and the tetramethylene (-CH₂-CH₂-CH₂-CH₂-) radical.

2-4C-Alkylene interrupted by oxygen stands for a straight chain alkylene radical having 1 to 4 carbon atoms which is suitably interrupted by an oxygen atom such as, for example, the [-CH₂-CH₂-O-CH₂-CH₂-] radical.

1-4C-Alkoxy represents radicals which, in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy and preferably the ethoxy and methoxy radicals.

1-4C-Alkoxy-1-4C-alkyl represents one of the abovementioned 1-4C-alkyl radicals, which is substituted by one of the abovementioned 1-4C-alkoxy radicals. Examples which may be mentioned are the methoxymethyl, the methoxyethyl and the isopropoxyethyl radicals, particularly the 2-methoxyethyl and the 2-isopropoxyethyl radicals.

1-4C-Alkoxy-2-4C-alkyl represents one of the abovementioned 2-4C-alkyl radicals, which is substituted by one of the abovementioned 1-4C-alkoxy radicals. Examples which may be mentioned are the methoxyethyl, ethoxyethyl and the isopropoxyethyl radicals, particularly the 2-methoxyethyl, the 2-ethoxyethyl and the 2-isopropoxyethyl radicals.

Hydroxy-2-4C-alkyl represents one of the abovementioned 2-4C-alkyl radicals, which is substituted by a hydroxy radical. An example which may be mentioned is the 2-hydroxyethyl or the 3-hydroxypropyl radical.

Phenyl-1-4C-alkyl stands for one of the abovementioned 1-4C-alkyl radicals, which is substituted by a phenyl radical. Examples which may be mentioned are the benzyl and the phenethyl radicals.

Halogen within the meaning of the invention is bromine or, in particular, chlorine or fluorine.

1-4C-Alkylcarbonyl represents a radical which, in addition to the carbonyl group, contains one of the abovementioned 1-4C-alkyl radicals. An example which may be mentioned is the acetyl radical.

1-4C-Alkylcarbonylamino represents an amino radical which is substituted by one of the abovementioned 1-4C-alkylcarbonyl radicals. An example which may be mentioned is the acetamido radical [CH₃C(O)-NH-].

1-4C-Alkylsulphonylamino is, for example, the propylsulfonylamino [C₃H₇S(O)₂NH-], the ethylsulfonylamino [C₂H₅S(O)₂NH-] and the methylsulfonylamino [CH₃S(O)₂NH-] radical.

HAR is a monocyclic or fused bicyclic 5- to 10-membered unsaturated heteroaromatic ring comprising one to three heteroatoms, each of which is selected from the group consisting of nitrogen, oxygen and sulphur.

In a first detail, HAR includes monocyclic 5-membered unsaturated heteroaromatic ring radicals comprising one, two or three heteroatoms, each of which is selected from the group consisting of nitrogen, oxygen and sulfur, such as e.g. thiophenyl, pyrrolyl, furanyl, oxazolyl, isoxazolyl, thiazolyl, isothiazolyl, imidazolyl, pyrazolyl, triazolyl (precisely: 1,2,4-triazolyl or 1,2,3-triazolyl), thiadiazolyl (precisely: 1,3,4-thiadiazolyl, 1,2,5-thiadiazolyl, 1,2,3-thiadiazolyl or 1,2,4-thiadiazolyl) or oxadiazolyl (precisely: 1,3,4-oxadiazolyl, 1,2,5-oxadiazolyl, 1,2,3-oxadiazolyl or 1,2,4-oxadiazolyl).

In a second detail, HAR includes monocyclic 6-membered unsaturated heteroaromatic ring radicals comprising one or two nitrogen atoms, such as e.g. pyridinyl, pyrimidinyl, pyrazinyl or pyridazinyl.

In a third detail, HAR includes fused bicyclic 9-membered unsaturated heteroaromatic ring radicals comprising one, two or three heteroatoms, each of which is selected from the group consisting of nitrogen, oxygen and sulfur, such as, for example, the benzo-fused derivatives of the aforementioned 5-membered monocyclic HAR radicals, such as e.g. benzothiophenyl, benzofuranyl, indolyl, benzoxazolyl, benzothiazolyl, indazolyl, benzimidazolyl, benzisoxazolyl, benzisothiazolyl, benzofurazanyl, benzotriazolyl, benzothiadiazolyl, isoindolyl, isofuranyl or isobenzothiophenyl, or indolizinyl.

In a fourth detail, HAR includes fused bicyclic 10-membered unsaturated heteroaromatic ring radicals comprising one or two heteroatoms, each of which is selected from the group consisting of nitrogen, oxygen and sulfur, such as, for example, the benzo-fused derivatives of the aforementioned 6-membered monocyclic HAR radicals, such as e.g. quinolinyl, isoquinolinyl, quinazolinyl, quinoxalinyl, phthalazinyl or cinnolinyl, or naphthyridinyl.

In a special detail, exemplary HAR radicals may include pyridinyl.

In another special detail, exemplary HAR radicals may include benzothiophenyl.

In another special detail, exemplary HAR radicals may include benzothiazolyl.

In another special detail, exemplary HAR radicals may include pyrazolyl.

In another special detail, exemplary HAR radicals may include imidazolyl.

In another special detail, exemplary HAR radicals may include thiazolyl.

It is to be stated, that the radical HAR is bonded via a ring carbon atom to the moiety T1.

Har1 is optionally substituted by R6111 and/or R6112, and is a monocyclic or fused bicyclic 5- to 10-membered unsaturated (heteroaromatic) heteroaryl radical comprising one to three heteroatoms, each of which is selected from the group consisting of nitrogen, oxygen and sulfur. In one detail, fused, in particular benzo-fused, bicyclic 9- or 10-membered heteroaryl radicals comprising one to three, in particular one or two, heteroatoms, each of which is selected from the group consisting of nitrogen, oxygen and sulfur, are to be mentioned.

Examples of Har1 include, withouit being restricted thereto, thiophenyl, furanyl, pyrrolyl, oxazolyl, isoxazolyl, pyrazolyl, imidazolyl, thiazolyl, isothiazolyl, triazolyl, oxadiazolyl, thiadiazolyl, pyridinyl, pyrimidinyl, pyrazinyl or pyridazinyl; and, in particular, the stable benzo-fused derivatives thereof, such as e.g. benzothiophenyl, benzofuranyl, indolyl, benzoxazolyl, benzothiazolyl, indazolyl, benzimidazolyl, benzisoxazolyl, benzisothiazolyl, benzofurazanyl, quinolinyl, isoquinolinyl, quinazolinyl, quinoxalinyl, phthalazinyl or cinnolinyl; and purinyl, indolizinyl, naphthyridinyl or pteridinyl.

In a special detail, exemplary Har1 radicals may include pyridinyl, benzimidazolyl, benzoxazolyl, benzofuranyl, benzothiophenyl and indolyl, such as e.g. pyridin-2-yl, pyridin-3-yl, pyridin-4-yl, benzimidazol-2-yl, benzoxazol-2-yl, benzofuran-2-yl, benzofuran-3-yl, benzothiophen-2-yl, benzothiophen-3-yl, indol-2-yl, indol-3-yl or indol-5-yl.

In a further special detail, an exemplary Har1 radical may be indolyl, such as e.g. indol-2-yl, indol-3-yl or indol-5-yl.

Yet in a further special detail, an exemplary Har1 radical may be pyridinyl, such as e.g. pyridin-2-yl, pyridin-3-yl or pyridin-4-yl.

As further examples of Har1, the R6111- and/or R6112-substituted derivatives of the abovementioned exemplary Har1 radicals may be mentioned.

Har1-1-4C-alkyl stands for one of the abovementioned 1-4C-alkyl radicals, such as e.g. methyl, ethyl or propyl, substituted by one of the abovementioned Har1 radicals, such as e.g. imidazolyl, benzimidazolyl, indolyl or pyrrolyl and the like or the substituted derivatives thereof. As examples may be mentioned, without being restricted thereto, pyridinylmethyl (e.g. pyridin-3-yl-methyl), imidazolylmethyl, pyrrolylmethyl, 2-imidazolylethyl (e.g. 2-imidazol-5-yl-ethyl), 2-pyridinylethyl, 3-(benzofuran-2-yl)propyl, 3-(benzimidazol-2-yl)propyl, 2-indolylethyl (e.g. 2-indol-2-yl-ethyl or 2-indol-3-yl-ethyl), indolylmethyl (e.g. indol-2-yl-methyl, indol-3-yl-methyl or indol-5-yl-methyl), 2-benzimidazolylethyl (e.g. 2-benzimidazol-2-ylethyl), benzimidazolylmethyl (e.g. benzimidazol-2-yl-methyl), and the like.

In a special detail, exemplary Har1-1-4C-alkyl radicals may include pyridinylmethyl (e.g. pyridin-3-yl-methyl, pyridin-4-yl-methyl or pyridin-4-yl-methyl), 2-pyridinylethyl (e.g. 2-pyridin-3-yl-ethyl), indolylmethyl (e.g. indol-2-yl-methyl, indol-3-yl-methyl or indol-5-yl-methyl) or 2-indolylethyl (e.g. 2-indolyl-2-yl-ethyl or 2-indolyl-3-yl-ethyl).

In a further special detail, exemplary Har1-1-4C-alkyl radicals may include pyridin-3-yl-methyl, pyridin-4-yl-methyl, 2-pyridin-3-yl-ethyl, indol-2-yl-methyl, indol-3-yl-methyl, indol-5-yl-methyl, 2-indolyl-2-yl-ethyl or 2-indolyl-3-yl-ethyl.

In the context of the radical Har1-1-4C-alkyl, it is to be stated, that the portion Har1 is bonded preferably via a ring carbon atom to the 1-4C-alkyl moiety.

One embodiment of those Har1-1-4C-alkyl radicals, in which the Har1 moiety is a fused bicyclic ring containing a benzene ring, refers to those radicals, in which the Har1 moiety is preferably bonded to the 1-4C-alkyl moiety via a carbon ring atom of the ring comprising one or more heteroatoms.

Another embodiment of those Har1-1-4C-alkyl radicals, in which the Har1 moiety is a fused bicyclic ring containing a benzene ring, refers to those radicals, in which the Har1 moiety is preferably bonded to the 1-4C-alkyl moiety via a carbon ring atom of the benzene ring.

Har2 stands for a monocyclic 5- or 6-membered unsaturated heteroaromatic ring comprising one to three heteroatoms, each of which is selected from the group consisting of nitrogen, oxygen and sulfur. Har2 may include, without being restricted thereto, thiophene, oxazole, isoxazole, thiazole, isothiazole, imidazole, pyrazole, triazole, thiadiazole, oxadiazole, pyridine, pyrimidine, pyrazine or pyridazine.

In a special detail, an exemplary Har2 radical may be pyridine.

Cyc1 stands for a ring system of formula la, which is bonded to the nitrogen atom of the carboxamide group via the moiety A. Cyc1 may include, without being restricted thereto, 2-aminophenyl substituted by R71 and/or R72.

In a special detail, an exemplary Cyc1 radical may be 2-aminophenyl.

Naphthyl, alone or as part of another group, includes naphthalen-1-yl and naphthalen-2-yl.

In the meaning of the present invention, it is to be understood, that, when two structural portions of the compounds according to this invention are linked via a constituent which has the meaning "bond", then said two portions are directly attached to another via a single bond.

As it is known for the skilled person, the expressions morpholino, 4N-(1-4C-alkyl)-piperazino, pyrrolidino and the like stand for morpholin-4-yl, 4N-(1-4C-alkyl)-piperazin-1-yl, pyrrolidin-1-yl and the like, respectively.

In general, unless otherwise mentioned the heterocyclic groups mentioned herein refer to all of the possible isomeric forms thereof.

The heterocyclic groups mentioned herein refer, unless otherwise noted, in particular to all of the possible positional isomers thereof.

Thus, for example, the term pyridyl or pyridinyl, alone or as part of another group, includes pyridin-2-yl, pyridin-3-yl and pyridin-4-yl, or, likewise, the term thiophenyl, alone or as part of another group, includes thiophen-2-yl or thiophen-3-yl.

Constituents which are optionally substituted as stated herein, may be substituted, unless otherwise noted, at any possible position.

The carbocyclic groups, alone or as part of other groups, mentioned herein may be substituted by their given substituents or parent molecular groups, unless otherwise noted, at any substitutable ring carbon atom.

The heterocyclic groups, alone or as part of other groups, mentioned herein may be substituted by their given substituents or parent molecular groups, unless otherwise noted, at any possible position, such as e.g. at any substitutable ring carbon or ring nitrogen atom.

Rings containing quaternizable imino-type ring nitrogen atoms (-N=) may be preferably not quatemized on these imino-type ring nitrogen atoms by the mentioned substituents or parent molecular groups.

Any heteroatom of a heterocyclic ring with unsatisfied valences mentioned herein is assumed to have the hydrogen atom(s) to satisfy the valences.

When any variable occurs more than one time in any constituent, each definition is independent.

Suitable salts for compounds of the formula I - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric- acid, malic acid such as (-)-L-malic acid or (+)-D-malic acid, fumaric acid, succinic acid, oxalic acid, tartaric acid such as (+)-L-tartaric acid or (-)-D-tartaric acid or meso-tartaric acid, embonic acid, stearic acid, toluenesulphonic acid, methanesulphonic acid or 3-hydroxy-2-naphthoic acid, the acids being employed in salt preparation - depending on whether a mono- or polybasic add is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds according to the invention on an industrial scale, are converted into pharmacologically tolerable salts by processes known to the person skilled in the art.

According to expert's knowledge the compounds of formula I of the invention as well as their salts may contain, e.g. when isolated in crystalline form, varying amounts of solvents. Included within the scope of the invention are therefore all solvates and In particular all hydrates of the compounds of formula I as well as all solvates and in particular all hydrates of the salts of the compounds of formula I.

In one embodiment of this invention, salts of the compounds of formula I include salts of compounds of formula I with hydrochloric acid.

The subststuents R61 and R62 of compounds of formula I can be attached in any possible position with respect to the binding position in which the AR ring is bonded to T1, whereby preference is given to the attachement of R61 and R62 to a ring carbon atom.

In one embodiment, AR is mono-substituted by R61. In another embodiment, AR is mono-substituted by R62. In another embodiment, AR is substituted by R61 and R62.

In a special embodiment, AR is mono-substituted by R61, and is pyridinyl, whereby particular emphasis Is given to the attachment of R61 in the meta or para position with respect to the binding position in which the pyridinyl ring Is bonded to the moiety T1. In a further special embodiment, AR is 6-(R61)-pyridin-3-yf.

In one embodiment, compounds according to the present invention more worthy to be mentioned are those compounds of formula I
in which
- R1: is hydrogen, or 1-4C-alkyl,
- R2: is hydrogen, or 1-4C-alkyl,
- R3: is hydrogen, or 1-4C-alkyl,
- R4: is hydrogen, or 1-4C-alkyl,
- R5: is hydrogen, or 1-4C-alkyl,
- R6: is -T1-Q1, in which
- T1: is a bond,
- Q1: is naphthyl, HAR, R61-substituted AR, R62-substituted AR, or R61- and R62-substituted AR, in which
- AR: is naphthyl, or HAR, in which
- HAR: is either
a monocyclic 5-membered unsaturated heteroaromatic ring comprising one, two or three heteroatoms, each of which is selected from the group consisting of nitrogen, oxygen and sulfur, or
a monocyclic 6-membered unsaturated heteroaromatic ring comprising one or two nitrogen atoms, or
a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,

- R61: is 1-4C-alkyl, or -T2-N(R611)R612, in which
- T2: is a bond or 1-4C-alkylene,
- R611: is hydrogen, 1-4C-alkyl, phenyl-1-4C-alkyl, or Har1-1-4C-alkyl, in which
- Har1: is either
a monocyclic 5-membered unsaturated heteroaromatic ring comprising one, two or three heteroatoms, each of which is selected from the group consisting of nitrogen, oxygen and sulfur, or
a monocyclic 6-membered unsaturated heteroaromatic ring comprising one or two nitrogen atoms, or
a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,
- R612: is hydrogen, 1-4C-alkyl, or hydroxy-2-4C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R62: is 1-4C-alkyl, 1-4C-alkoxy, or halogen,
- R7: is hydroxyl, or 2-aminophenyl,
and the salts of these compounds.

In another embodiment, compounds according to the present invention more worthy to be mentioned are those compounds of formula I
in which
- R1: is hydrogen, or 1-4C-alkyl,
- R2: is hydrogen, or 1-4C-alkyl,
- R3: is hydrogen, or 1-4C-alkyl,
- R4: is hydrogen, or 1-4C-alkyl,
- R5: is hydrogen, or 1-4C-alkyl,
- R6: is -T1-Q1, in which
- T1: is a bond,
- Q1: is naphthyl, HAR, R61-substituted AR, or R62-substituted AR, in which
- AR: is naphthyl, or HAR, in which
- HAR: is either
a monocyclic 5-membered unsaturated heteroaromatic ring comprising one, two or three heteroatoms, each of which is selected from the group consisting of nitrogen, oxygen and sulfur, or
a monocyclic 6-membered unsaturated heteroaromatic ring comprising one or two nitrogen atoms, or
a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,
- R61: is 1-4C-alkyl, or -T2-N(R611)R612, in which
- T2: is a bond or 1-4C-alkylene,
- R611: is hydrogen, 1-4C-alkyl, phenyl-1-4C-alkyl, or Har1-1-4C-alkyl, in which
- Har1: is either
a monocyclic 5-membered unsaturated heteroaromatic ring comprising one, two or three heteroatoms, each of which is selected from the group consisting of nitrogen, oxygen and sulfur, or
a monocyclic 6-membered unsaturated heteroaromatic ring comprising one or two nitrogen atoms, or
a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,
- R612: is hydrogen, 1-4C-alkyl, or hydroxy-2-4C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R62: is 1-4C-alkyl, 1-4C-alkoxy, or halogen,
- R7: is hydroxyl, or 2-aminophenyl,
and the salts of these compounds.

In one embodiment, compounds according to the present invention in particular worthy to be mentioned are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, R61-substituted AR, N-methyl-imidazolyl, N-methyl-pyrazolyl, N-methyl- indolyl, mono- or di-methyl-substituted thiazolyl, methyl -substituted N-methyl-imidazolyl, or methyl substituted N-methyl-pyrazolyl, in which
- AR: is naphthyl, or HAR, in which
- HAR: is pyridinyl, thiazolyl, benzothiophenyl, benzothiazolyl, benzofuranyl, indolyl, quinolinyl or isoquinolinyl,
- R61: is 1-4C-alkyl, or -T2-N(R611)R612. in which
- T2: is a bond or 1-2C-alkylene,
- R611: is hydrogen or 1-4C-alkyl,
- R612: is hydrogen or 1-4C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R7: is hydroxyl, or 2-aminophenyl,
and the salts of these compounds.

In another embodiment, compounds according to the present invention in particular worthy to be mentioned are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, R61-substituted pyridinyl, or N-methyl-indolyl, in which
- HAR: is pyridinyl, benzothiophenyl, benzofuranyl, indolyl, quinolinyl or isoquinolinyl,
- R61: is 1-4C-alkyl, or -T2-N(R611)R612, in which
- T2: is a bond or 1-2C-alkylene,
- R611: is hydrogen or 1-4C-alkyl,
- R612: is hydrogen or 1-4C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R7: is hydroxyl, or 2-aminophenyl,
and the salts of these compounds.

In one embodiment, compounds according to the present invention in more particular worthy to be mentioned are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, R61-substituted pyridinyl, N-methyl-imidazolyl, N-methyl-pyrazolyl, mono- or di-methyl-substituted thiazolyl, methyl -substituted N-methyl-imidazolyl, or methyl substituted N-methyl-pyrazolyl, in which
- HAR: is pyridinyl, thiazolyl, benzothiophenyl or benzothiazolyl,
- R61: is -T2-N(R611)R612, in which
- T2: is a bond or 1-2C-alkylene,
- R611: is hydrogen or 1-2C-alkyl,
- R612: is hydrogen or 1-2C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R7: is hydroxyl, or 2-aminophenyl,
and the salts of these compounds.

In another embodiment, compounds according to the present invention in more particular worthy to be mentioned are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, or R61-substituted pyridinyl, in which
- HAR: is pyridinyl,
- R61: is -T2-N(R611)R612, in which
- T2: is a bond or 1-2C-alkylene,
- R611: is hydrogen or 1-2C-alkyl,
- R612: is hydrogen or 1-2C-alkyl,
- or R611 and R612: together and with inclusion of the nitrogen atom, to which they are bonded, form a heterocyclic ring Het1, in which
- Het1: is morpholino, piperidino, pyrrolidino, piperazino, or 4N-methyl-piperazino,
- R7: is hydroxyl, or 2-aminophenyl,
and the salts of these compounds.

In one embodiment, compounds according to the present invention to be emphasized are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, 2-(R61)-pyridin-3-yl, 1-methyl-pyrazol-4-yl, 1-methyl-imidazol-4-yl, 1,2- dimethyl-imidazol-4-yl, or 2,4-dimethyl-thiazol-5-yl, in which
- HAR: is pyridin-3-yl, benzothiophen-2-yl or benzothiazol-6-yl,
- R61: is -T2-N(R611)R612, in which
- T2: is a bond or methylene,
- R611: is hydrogen or methyl,
- R612: is hydrogen or methyl,
- or R611 and R612: together and with inclusion of the nitrogen atom, to which they are bonded, form a heterocyclic ring Het1. in which
- Het1: is morpholino,
- R7: is hydroxyl,
and the salts of these compounds.

In a further embodiment, compounds according to the present invention to be emphasized are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, 2-(R61)-pyridin-3-yl, 1-methyl-pyrazol-4-yl, 1-methyl-imidazol-4-yl, 1,2- dimethyl-imidazol-4-yl, or 2,4-dimethyl-thiazol-5-yl, in which
- HAR: is pyridin-3-yl, benzothiophen-2-yl or benzothiazol-6-yl,
- R61: is -T2-N(R611)R612, in which
- T2: is a bond or methylene,
- R611: is hydrogen or methyl,
- R612: is hydrogen or methyl,
- or R611 and R612: together and with inclusion of the nitrogen atom, to which they are bonded, form a heterocyclic ring Het1. in which
- Het1: is morpholino,
- R7: is 2-aminophenyl,
and the salts of these compounds.

In another embodiment, compounds according to the present invention to be emphasized are those compounds of formula I
in which
- R1: is hydrogen,
- R2: is hydrogen,
- R3: is hydrogen,
- R4: is hydrogen,
- R5: is hydrogen,
- R6: is -T1-Q1, in which
- T1: is a bond,
- Q1: is naphthyl, HAR, or 2-(R61)-pyridin-3-yl, in which
- HAR: is pyridinyl,
- R61: is -T2-N(R611)R612, in which
- T2: is a bond or methylene,
- R611: is hydrogen or methyl,
- R612: is hydrogen or methyl,
- or R611 and R612: together and with inclusion of the nitrogen atom, to which they are bonded, form a heterocyclic ring Het1, in which
- Het1: is morpholino,
- R7: is hydroxyl, or 2-aminophenyl,
and the salts of these compounds.

A special interest in the compounds according to the present invention refers to those compounds of this invention which are included -within the scope of this invention- by one or, when possible, a combination of more of the following embodiments:
An embodiment of the compounds according to the present invention relates to those compounds of formula I, in which R1, R2, R3, R4 and R5 are all hydrogen.
A further embodiment of the compounds according to the present invention relates to those compounds of formula I, in which R7 is hydroxyl.
A further embodiment of the compounds according to the present invention relates to those compounds of formula I, in which R7 is 2-aminophenyl.
A further embodiment of the compounds according to the present invention relates to those compounds of formula I, in which R7 is aminopyridyl.
A further embodiment of the compounds according to the present invention relates to those compounds of formula I, in which R7 is Cyc1, whereby in a subembodiment thereof Cyc1 is 2-phenyl.
A further embodiment of the compounds according to the present invention relates to those compounds of formula I, in which T1 is a bond.
A further embodiment of the compounds according to the present invention relates to those compounds of formula I, in which R6 is naphthyl.
A further embodiment of the compounds according to the present invention relates to those compounds of formula I, in which R6 is HAR.
A further embodiment of the compounds according to the present invention relates to those compounds of formula I, in which R6 is benzothiophenyl or benzothiazolyl.
A further embodiment of the compounds according to the present invention relates to those compounds of formula I, in which R6 is R61- and/or R62-substituted HAR.
A further embodiment of the compounds according to the present invention relates to those compounds of formula I, in which R6 is R61-substituted HAR.
A further embodiment of the compounds according to the present invention relates to those compounds of formula I, in which R6 is R61-substituted pyridinyl.
A further embodiment of the compounds according to the present invention relates to those compounds of formula I, in which R6 is 6-(R61)-pyridin-3-yl.
A further embodiment of the compounds according to the present invention relates to those compounds of formula I, in which R6 is N-methyl-imidazolyl, N-methyl-pyrazolyl, mono- or di-methyl-substituted thiazolyl, methyl -substituted N-methyl-imidazolyl, or methyl substituted N-methyl-pyrazolyl.
A further embodiment of the compounds according to the present invention relates to those compounds of formula I, in which T2 is a bond.
A further embodiment of the compounds according to the present invention relates to those compounds of formula I, in which T2 is 1-4C-alkylene, such as e.g. 1-2C-alkylene.
A further embodiment of the compounds according to the present invention relates to those compounds of formula I, in which R1, R2, R3, R4 and R5 are all hydrogen, and R7 is hydroxyl.
A further embodiment of the compounds according to the present invention relates to those compounds of formula I, in which R1, R2, R3, R4 and R5 are all hydrogen, and R7 is 2-aminophenyl.

It is to be understood, that the present invention also includes any or all possible combinations and subsets of the embodiments defined herein afore.

Exemplary compounds according to this invention may include any one selected from
(E)-N-Hydroxy-3-[1-(naphthalene-2-sulfonyl)-1H-pyrrol-3-yl]-acrylamide,
(E)-N-(2-Amino-phenyl)-3-[1-(naphthalene-2-sulfonyl)-1H-pyrrol-3-yl]-acrylamide,
(E)-N-Hydroxy-3-[1-(pyridine-3-sulfonyl)-1H-pyrrol-3-yl]-acrylamide,
(E)-N-Hydroxy-3-[1-(6-morpholin-4-yl-pyridine-3-sulfonyl)-1H-pyrrol-3-yl]-acrylamide,
(E)-N-(2-Amino-phenyl)-3-[1-(benzo[b]thiophene-2-sulfonyl)-1H-pyrrol-3-yl]-acrylamide,
(E)-N-(2-Amino-phenyl)-3-[1-(benzothiazole-6-sulfonyl)-1H-pyrrol-3-yl]-acrylamide,
(E)-N-Hydroxy-3-[1-(1-methyl-1H-imidazole-4-sulfonyl)-1H-pyrrol-3-yl]-acrylamide,
(E)-N-Hydroxy-3-[1-(1-methyl-1H-pyrazole-4-sulfonyl)-1H-pyrrol-3-yl]-acrylamide,
(E)-3-[1-(Benzo[b]thiophene-2-sulfonyl)-1H-pyrrol-3-yl]-N-hydroxy-acrylamide,
(E)-3-[1-(Benzothiazole-6-sulfonyl)-1H-pyrrol-3-yl]-N-hydroxy-acrylamide,
(E)-3-[1-(2,4-Dimethyl-thiazole-5-sulfonyl)-1H-pyrrol-3-yl]-N-hydroxy-acrylamide, and
(E)-3-[1-(1,2-Dimethyl-1H-imidazole-4-sulfonyl)-1H-pyrrol-3-yl]-N-hydroxy-acrylamide,
and the salts thereof.

The compounds according to the present invention can be prepared, for example, as shown in the reaction schemes below and according to the reaction steps specified as follows, or, particularly, in a manner as described by way of example in the following examples, or analogously or similarly thereto using preparation procedures and synthesis strategies known to the person skilled in the art.

In reaction scheme 1 the carbon chain of compounds of formula V, in which R1, R2, R4 and R5 have the meanings mentioned above, is lengthened, for example, by a condensation reaction (with a malonic acid derivative) or by a Wittig or Julia reaction or, particularly in the case when R2 is hydrogen, by a Horner-Wadsworth-Emmons reaction (with a β-(alkoxycarbonyl)-phosphonic acid dialkyl ester) to obtain compounds of formula IV, in which R1, R2, R3, R4 and R5 have the meanings mentioned above and PG1 stands for a suitable temporary protective group for the carboxyl group, for example tert-butyl or one of those art-known protective groups mentioned in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000). Compounds of formula V, in which R1, R2, R4 and R5 have the meanings mentioned above, are known, or can be prepared according to art-known procedures, or can be obtained as described in the following examples for the case that R2 is hydrogen from compounds of formula VI.

Compounds of formula VI are known or are accessible in a known manner or as described in the following examples.

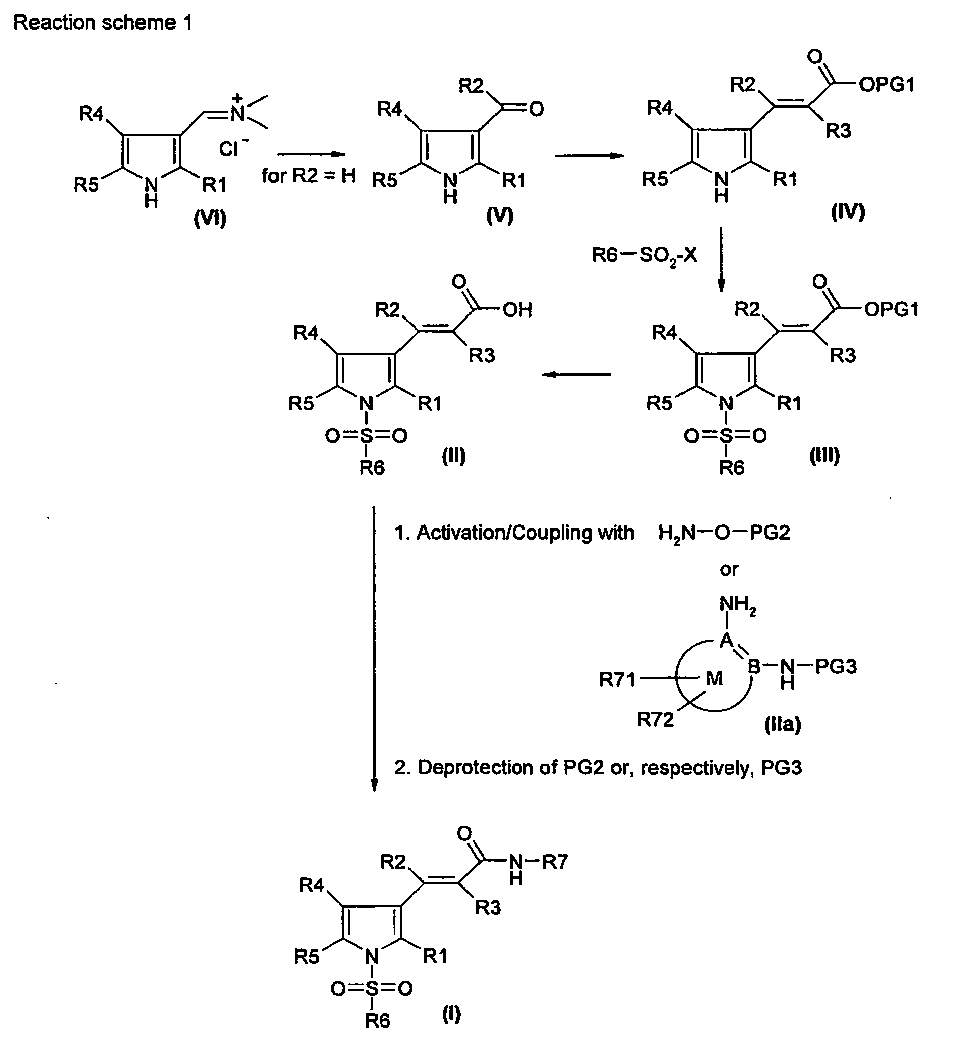

Compounds of formula IV, in which R1, R2, R3, R4 and R5 have the meanings mentioned above and PG1 stands for a said suitable protective group, can be reacted with compounds of formula R6-SO₂X, in which R6 has the meanings mentioned above and X is a suitable leaving group, such as e.g. chlorine, to give the corresponding compounds of formula III.

In the next reaction step, the protective group PG1 of compounds of formula III can be removed in a manner as described in the following examples or according to an art-known manner to afford compounds of formula II.

Compounds of formula R6-SO₂-X are known or can be prepared in a known manner.

Compounds of formula II, in which R1, R2, R3, R4, R5 and R6 have the meanings given above, can be coupled with compounds of formulae H₂N-O-PG2, in which PG2 is a suitable oxygen protective group such as e.g. a suitable silyl or tetrahydropyran-2-yl protective group, or IIa, in which PG3 stands for a suitable nitrogen protective group such as e.g. the tert-butyloxycarbonyl protective group, by reaction with amide bond linking reagents optionally in the presence of coupling additives known to the person skilled in the art. Exemplary amide bond linking reagents known to the person skilled in the art which may be mentioned are, for example, carbodiimides (e.g. dicyclohexylcarbodiimide or, preferably, 1-ethyl-3-(3-dimethylaminopropyl)carbodiimide hydrochloride), azodicarboxylic acid derivatives (e.g. diethyl azodicarboxylate), uronium salts [e.g. O-(benzotriazol-1-yl)-N,N,N',N'-tetramethyluronium tetrafluoroborate or O-(benzotriazol-1yl)-N,N,N',N'-tetramthyl-uronium-hexafluorophosphate] and N,N'-carbonyldiimidazole.

Alternatively, compounds of formula II can be activated prior to the coupling reaction by forming an acid halide or acid anhydride optionally in an in-situ procedure without isolating the acid halide or acid anhydride.

Compounds of formulae H₂N-O-PG2 or IIa are known or can be prepared according to art-known processes.

Removal of the protective groups PG2 or PG3 can be obtained in a manner known for the person skilled in the art or as described in the following examples to give compounds of formula I, in which R1, R2, R3, R4, R5, R6 and R7 have the meanings mentioned above.

In an alternative synthesis route, the activation/coupling with compounds of formulae H₂N-O-PG2 or IIa may be done prior to the reaction with compounds of formula R6-SO₂-X

Compounds of formula I, in which T2 is 1-4C-alkylene, particularly methylene, can be prepared as outlined in the following reaction schemes 2 to 5, and specified below, or as described by way of example in the following examples, or analogously or similarly thereto.

As shown in reaction scheme 2 compounds of formula VII, in which AR has the meanings given above, T2 is 1-4C-alkylene, particularly methylene, and Y1 is a suitable leaving group, such as e.g. iodine, chlorine or, particularly, bromine, and PG4 stands for a suitable temporary protective group for the carboxyl group, for example tert-butyl, can be reacted with compounds of formula HN(R611)R612 to give in an art-known nucleophilic substitution reaction corresponding amino compounds, which are deprotected by removal of PG4 to give corresponding free acids of formula VIII, which can be coupled with compounds of formulae H₂N-O-PG2 or IIa as described above to give, after removal of PG2 or PG3, corresponding compounds of formula Ii.

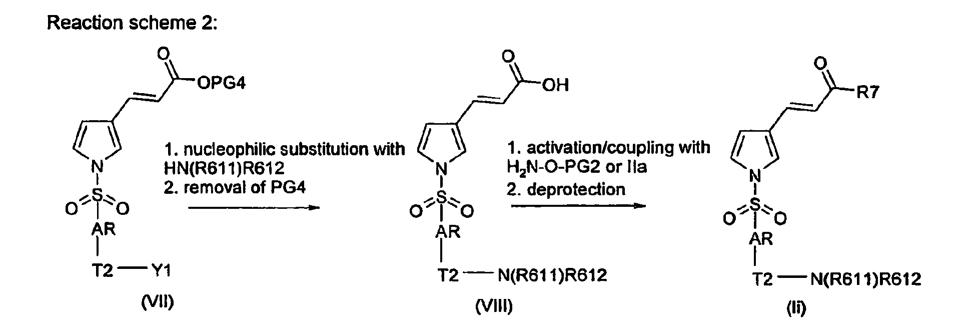

Alternatively, as shown in reaction scheme 3, compounds of formula VII, in which AR has the meanings given above, T2 is 1-4C-alkylene, particularly methylene, and Y1 is a suitable leaving group, such as e.g. iodine, chlorine or, particularly, bromine, and PG4 stands for a suitable temporary protective group for the carboxyl group, for example tert-butyl, can be reacted with a temporarily protected amine (a primary or, particularly, a secondary one), such as e.g. phthalimide, to give in an art-known nucleophilic substitution reaction corresponding amino compounds, which are deprotected by removal of PG4 to give corresponding free acids of formula IX, which can be coupled with compounds of formulae H₂N-O-PG2 or IIa as described above to give corresponding compounds of formula X.

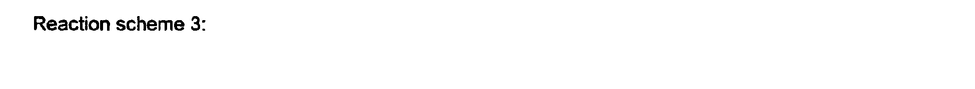

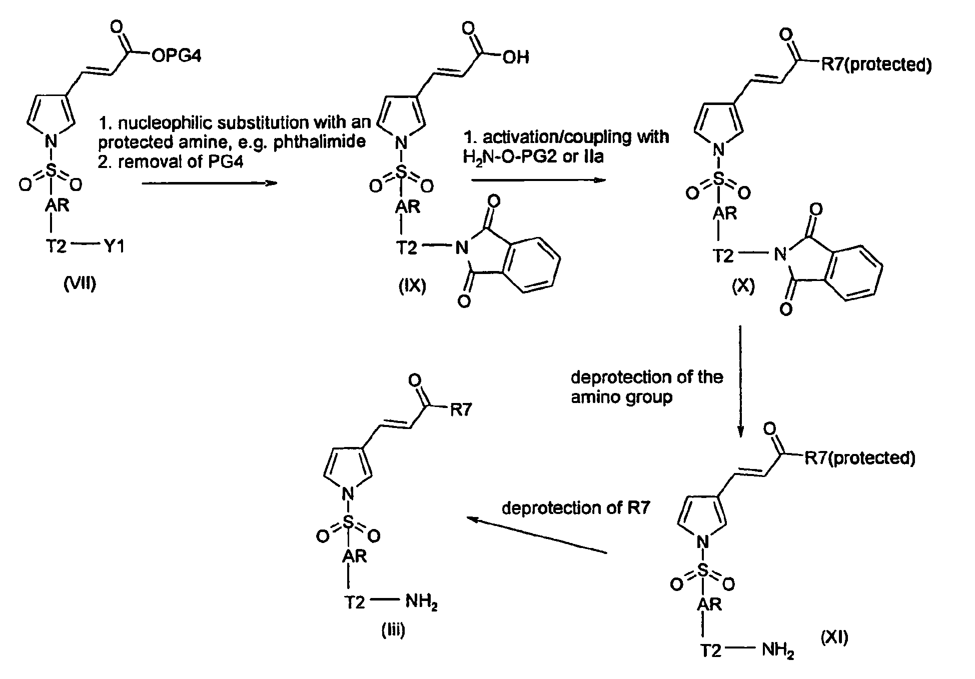

The amino moiety of compounds of formula X can be deprotected in an art-known manner to give corresponding compounds of formula XI, such as e.g. when the phthalimido protective group is used, this can be removed in a manner customary per se to the skilled person, e.g. with the aid of hydrazine.

Compounds of formula XI can be deprotected to give corresponding compounds of formula Iii.

Alternatively, as shown in reaction scheme 4, compounds of formula XI can be reacted with compounds of formula R611-Y1 and/or R612-Y2, in which R611 and R612 have the meanings given above and are different from hydrogen and Y1 and Y2 are suitable leaving groups such as e.g. chlorine, bromine, iodine or a sulfonate (e.g. triflate) leaving group, to give in an art-known nucleophilic substitution reaction corresponding compounds of formula XII or XII'. Compounds of formula XII or XII' can be deprotected to give corresponding compounds of formula Iiii or Iiv, respectively.

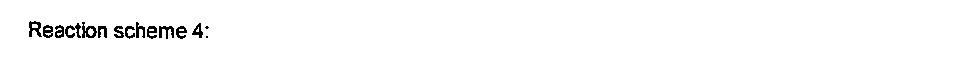

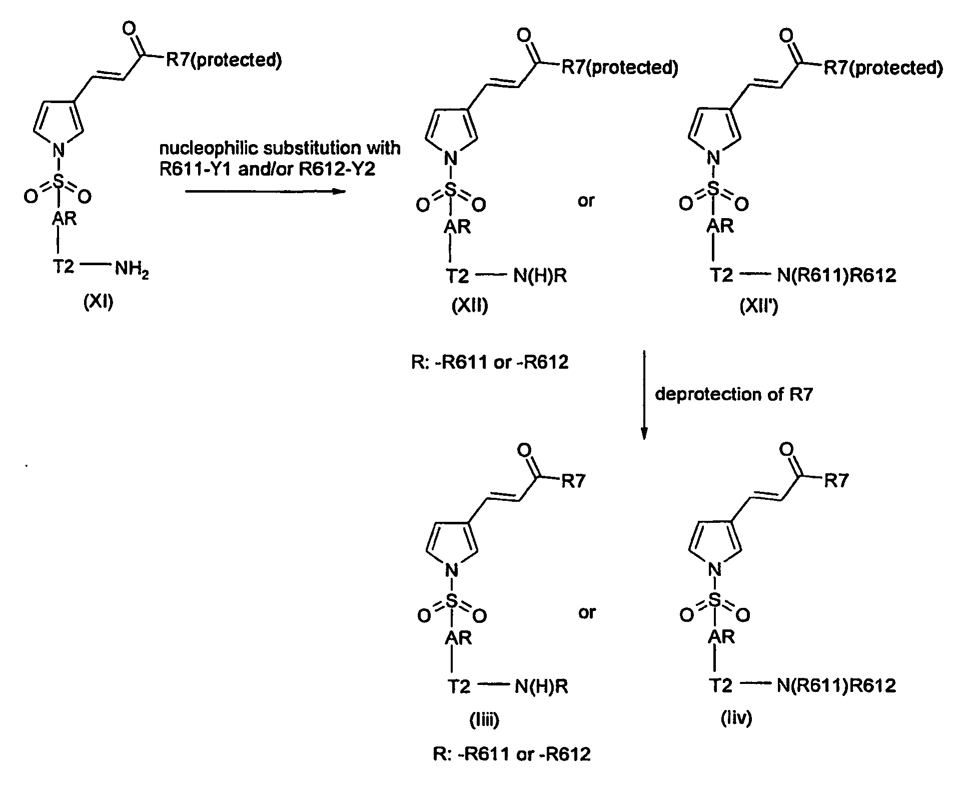

Yet alternatively, as shown in reaction scheme 5, compounds of formula XI can be reacted with aldehydes or ketones in an reductive amination reaction, such as e.g. compounds of formula XI can be reacted with benzaldehyde, or compounds of formulae 1-3C-alkyl-CHO or Har1-CHO, in which Har1 has the meanings given above, to give in an art-known reductive amination reaction corresponding compounds of formula XIII. Compounds of formula XIII can be deprotected to give corresponding compounds of formula Iv.

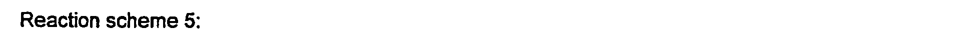

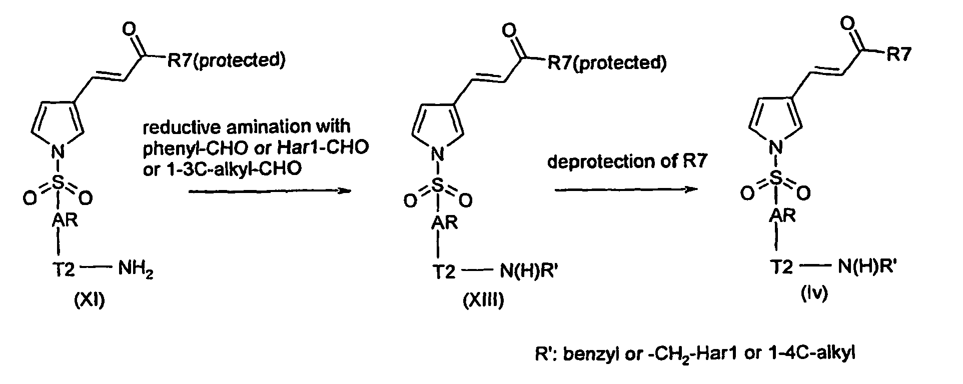

Starting from the appropriate starting compounds, compounds of formula VII can be obtained according to the synthesis route shown in reaction scheme 1 and described above, according to art-known procedures, or analogously or similarly thereto.

The abovementioned compounds of formulae HN(R611)R612, R611-Y1, R612-Y2, 1-3C-alkyl-CHO or Har1-CHO are known or can be obtained according to art-known procedures.

When the protective groups PG2 or PG3 are deprotected or purification is carried out under the presence of an inorganic or organic acid (e.g. hydrochloric acid or formic acid), the compounds of formula I may be obtained -depending on their individual chemical nature and the individual nature of the acid used- as free base or containing said acid in an stoechiometric or non-stoechiometric quantity. The amount of the acid contained can be determined according to art-known procedures, e.g. by titration.

When the compounds of formula I are chiral compounds (e.g. by having one or more chiral centers), the invention refers to all conceivable stereoisomers, like e.g. diastereomers and enantiomers, in substantially pure form as well as in any mixing ratio, including the racemates, as well as the salts thereof.

In general, enantiomerically pure compounds of this invention may be prepared according to art-known processes, such as e.g. via asymmetric syntheses using chiral synthons or chiral reagents; by chromatographic separation on chiral separating columns; by means of salt formation of the racemic compounds with optically active acids or bases, subsequent resolution of the salts and release of the desired compound from the salt; by derivatization with chiral auxiliary reagents, subsequent diastereomer separation and removal of the chiral auxiliary group; or by (fractional) crystallization from a suitable solvent.

The reactions mentioned above can be expediently carried out analogously to the methods known to the person skilled in the art or as described by way of example in the following examples.

It is moreover known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups Is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series) by P. Kocienski (Thieme Medical Publishers, 2000).

The isotation and purification of the substances according to the invention is carried out in a manner known per se, e.g, by distilling off the solvent in vacuo and recrystallizing the resulting residue from a suitable solvent or subjecting it to one of the customary purification methods, such as, for example, column chromatography on suitable support materiaL

Optionally, compounds of formula I can be converted into their salts, or, optionally, salts of the compounds of formula I can be converted into the free compounds of formula I.

Salts are obtained by dissolving the free compound In a suitable solvent (e.g. a ketone, such as acetone, methyl ethyl ketone or methyl isobutyl ketone, an ether, such as diethyl ether, tetrahydrofuran or dioxane, a chlorinated hydrocarbon, such as methylene chloride or chloroform, or a low molecular weight aliphatic alcohol such as methanol, ethanol or isopropanol which contains the desired acid or base, or to which the desired acid or base is then added. The salts are obtained by filtering, reprocipitating, precipitating with a nonsolvent for the addition salt or by evaporating the solvent Salts obtained can be converted by alkalization or by acidification into the free compounds, which In turn can be converted into salts. In this way, pharmacologically intolerable salts can be converted into pharmacologically tolerable salts.

Suitably, the conversions mentioned in this invention can be carried out analogously or similarly to methods which are familiar per se to the person skilled in the art

The person skilled In the art knows on the basis of his/her knowledge and on the basis of those synthesis routes, which are shown and described within the description of this invention, how to find other possible synthesis routes for compounds of the formula I.

The following examples serve to illustrate the invention further without restricting it Likewise, further compounds of the formula I including their salts, whose preparation is not explicitly described, can be prepared in an analogous manner or In a manner familiar per se to the person skilled In the art using customary process techniques.

Any or all of the compounds of formula I which are mentioned as final products in the following examples as well as their salts are a preferred subject of the present invention.

In the examples, MS stands for mass spectrum, M for molecular ion, TSP for Thermospray Ionization, ESI for Electrospray ionization, EI for Electron Ionization, h for hours, min for minutes. Other abbreviations used herein have the meanings customary per se to the person skilled in the art.

### Examples

### Final products

### 1. (E)-N-Hydroxy-3-[naphthalene-2-sulfonyl)-1H-pyrrol-3-yl]-acrylamide

To a mixture of 0.5 g (E)-3-[1-(Naphthalene-2-sulfonyl)-1H-pyrrol-3-yl]-N-(tetrahydro-pyran-2-yloxy)-acrylamide (compound A1) in 2 ml methanol are added 11.3 ml 0.5M aqueous HCl and the reaction mixture is stirred overnight. The resulting suspension is diluted with methanol and the mixture is further reacted at 4 °C for 3 days. The resulting solid is separated and the residue is crystallized by means of dichloromethane. A nearly colorless solid is obtained with a melting point of 152.8 °C.

### 2. (E)-N-(2-Amino-phenyl)-3-[1-(naphthalene-2-sulfonyl)-1H-pyrrol-3-yl]-acrylamide

10 ml of a 33% solution of HBr in acetic acid are cooled to -10 °C. To this solution is added a solution of 0.2 g (2-{(E)-3-[1-(Naphthalene-2-sulfonyl)-1H-pyrrol-3-yl]-allanoylamino}-phenyl)-carbamic acid tert-butyl ester (compound A2) in 2 ml ethyl acetate. After stirring of this mixture at -10 °C the reaction is quenched with 20 ml of a 5% aqueous solution of sodium bicarbonate. The resulting mixture is extracted with 25 ml ethyl acetate, the organic phases are dried and evaporated. The crude product is crystallized from dichloromethane.
m.p.: 186.6°C

### 3. (E)-N-Hydroxy-3-[1-(pyridine-3-sulfonyl)-1H-pyrrol-3-yl]-acrylamide

To a mixture of 38mg (E)-3-[1-(Pyridine-3-sulfonyl)-1H-pyrrol-3-yl]-N-(tetrahydro-pyran-2-yloxy)-acrylamide (compound A3) in 1 ml methanol are added 5 ml 0.1M aqueous HCl and the mixture is stirred overnight. The mixture is evaporated and the residue crystallized from water. The compound may contain HCl.
mp: 161.4-176.4°C

### 4. (E)-N-Hydroxy-3-[1-(6-morpholin-4-yl-pyrldine-3-sulfonyl)-1H-pyrrol-3-yl]-acrylamide

The title compound is prepared analogously to Example 1. The title compound is isolated as brownish oil.

### 5. (E)-N-(2-Amino-phenyl)-3-[1-(benzo[b]thiophene-2-sulfonyl)-1H-pyrrol-3-yl]-acrylamide, compound with hydrochloric acid

78 mg (2-{(E)-3-[1-(Benzo[b]thiophene-2-sulfonyl)-1-H-pyrrol-3-yl]-allanoylamino}-phenyl)-carbamic acid tert-butyl ester are suspended in 5 ml 4 M HCl in dioxane. The suspension is stirred overnight at ambient temperature. The solvents are evaporated and the residue is dissoved in acetonitrile/water and lyophilized. By this method 65 mg of a yellow solid are obtained. The compound contains HCl. The compound sinters at 101°C.

### 6. (E)-N-(2-Amino-phenyl)-3-[1-(benzothiazole-6-sulfonyl)-1H-pyrrol-3-yl]-acrylamide, compound with hydrochloric acid

267 mg (2-{(E)-3-[1-(Benzothiazole-6-sulfonyl)-1H-pyrrol-3-yl]-allanoytamino}-phenyl)-carbamic acid tert-butyl ester are suspended in 18 ml 4 M HCl in dioxane. The suspension is stirred overnight at ambient temperature. Overnight a pink suspension is formed. Solvents are evaporated, the residue collected and washed with diisopropylether. The product is dried in high vacuo. By this method 201 mg of a beige solid are obtained. The compound contains HCl. The compound decomposes at 161 °C.

### 7. (E)-N-Hydroxy-3-[1-(1-methyl-1H-imidazole-4-sulfonyl)-1H-pyrrol-3-yl]-acrylamide, compound with hydrochloric acid

90 mg (E)-3-[1-(1-Methyl-1H-imidazole-4-sulfonyl)-1H-pyrrol-3-yl]-N-(tetrahydro-pyran-2-yloxy)-acrylamide are dissolved in 3 ml methanol. 11 ml 1 M aqueous HCl are added and the solution is stirred at ambient temperature for 24 h. Solvents are evaporated, water is added and the product is lyophilized. The residue is washed with ethylacetate. By this method 10 mg of a white solid are obtained. The compound contains HCl. Melting point is at 154-158°C.

### 8. (E)-N-Hydroxy-3-[1-(1-methyl-1H-pyrazole-4-sulfonyl)-1H-pyrrol-3-yl]-acrylamide, compound with hydrochloric acid

79 mg (E)-3-[1-(1-Methyl-1H-pyrazole-4-sulfonyl)-1H-pyrrol-3-yl]-N-(tetrahydro-pyran-2-yloxy)-acrylamide are solved in 3 ml methanol. By adding 9 ml 1 M aqueous HCl a solid precipitates and the solution is stirred at ambient temperature for 24 h. The colour changes from yellow to pink. Solvents are evaporated, the residue is washed with ethylacetate and dried in vacuo. By this method 23 mg of a pink solid are obtained. The compound contains HCl. The melting point is at 163-166°C.

### 9. (E)-3-[1-(Benzo[b]thiophene-2-sutfonyl)-1H-pyrrol-3-yl]-N-hydroxy-acrylamide

196 mg (E)-3-[1-(Benzo[b]thiophene-2-sulfonyl)-1H-pyrrol-3-yl]-N-(tetrahydro-pyran-2-yloxy)-acrylamide are solved in 5 ml methanol. By adding 17 ml 1 M aqueous HCl a solid precipitates and the solution is stirred at ambient temperature for 24 h. The suspension is filtered and the residue is washed with water, ethylacetate and dried in vacuo. By this method 121 mg of a white solid are obtained. The compound might contain some HCl. The melting point is at 186-188°C.

### 10. (E)-3-[1-(Benzothiazole-6-sulfonyl)-1H-pyrrol-3-yl]-N-hydroxy-acrylamide

190 mg (E)-3-[1-(Benzothiazole-6-sulfonyl)-1H-pyrrol-3-yl]-N-(tetrahydro-pyran-2-yloxy)-acrylamide are dissolved in 7 ml methanol. By adding 20 ml 1 M aqueous HCl a solid precipitates and the solution is stirred at ambient temperature for 24 h. The suspension is filtered and the residue is washed with water, ethylacetate and dried in vacuo. The crude product is purified by HPLC. The compound might contain formic acid from the HPLC-buffer. By this method 87 mg of a white solid are obtained. Melting point is at 225-229°C.

### 11. (E)-3-[1-(2,4-Dimethyl-thiazole-5-sulfonyl)-1H-pyrrol-3-yl]-N-hydroxy-acrylamide, compound with hydrochloric acid

200 mg (E)-3-[1-(2,4-Dimethyl-thiazole-5-sulfonyl)-1H-pyrrol-3-yl]-N-(tetrahydro-pyran-2-yloxy)-acrylamide are dissolved in 6 ml methanol. By adding 19 ml 1 M aqueous HCl a solid precipitates and the solution is stirred at ambient temperature for 24 h. The solid is filtered and washed with ethylacetate and dried in vacuo. By this method 124 mg of a white solid are obtained. The compound contains HCl. Melting point: 181-182 °C

### 12. (E)-3-[1-(1,2-Dimethyl-1H-imidazote-4-sulfonyl)-1H-pyrrol-3-yl]-N-hydroxy-acrylamide

64 mg (E)-3-[1-(1,2-Dimethyl-1H-imidazole-4-sulfonyl)-1H-pyrrol-3-yl]-N-(tetrahydro-pyran-2-yloxy)-acrylamide are solved in 2 ml methanol. By adding 5 ml 1 M aqueous HCl a solid precipitates. The solution is stirred at ambient temperature for 24 h. The red solution is lyophilized and purified by silica gel flash chromatography. By this method 5 mg of a bright beige solid are obtained. Melting point 190-194°C

Using similar procedures to those described herein, but with suitable choice of starting materials, further compounds according to this invention may be prepared.

### Starting materials

### A1. (E)-3-[1-(Naphthalene-2-sulfonyl)-1H-pyrrol-3-yl]-N-(tetrahydro-pyran-2-yloxy)-acrylamide

To a solution of 0.863 g (E)-3-[1-(Naphthalene-2-sulfonyl)-1H-pyrro)-3-yl]-acrylic acid (compound B1) in 50 ml DMF are added 0.404 g N-hydroxybenzotriazole hydrate (HOBtxH2O) and 2.67g triethylamine. This mixture is stirred for 30 min at ambient temperature and then are added 1.52g 1-ethyl-3-(3-dimethyl aminopropyl)-carbodiimide hydrochloride (EDCxHCl) and the mixture is stirred for further 45 min. Now 0.309 g O-(Tetrahydro-2H-pyran-2-yl)-hydroxylamine are added and the reaction mixture is stirred 4h at ambient temperature. The solvent is removed in vacuo and the residue is partitioned between ethyl acetate and water. The water phase is extracted twice with ethyl acetate and the combined organic phases are dried and evaporated. The crude product is purified by means of silica gel flash chromatography. 1.03 g of a nearly colorless foam is obtained.

### A2. (2-{(E)-3-[1-(Naphthalene-2-sulfonyl)-1H-pyrrol-3-yl]-allanoylamino}-phenyl)-carbamic acid tert-butyl ester

A mixture of 0.5 g (E)-3-[1-(Naphthalene-2-sulfonyl)-1H-pyrrol-3-yl]-acrylic acid (compound B1), 30 ml DMF, 0.234 g WOBtxH2O and 1.54 g triethylamine is stirred at ambient temperature for 30 min. Now 0.88 g EDCxHCl are added and the mixture is stirred for 45 min at ambient temperature. Then 0.319 g N-boc-o-phenylenediamine are added and the reaction mixture is stirred for 19.5 h. The reaction mixture is evaporated under high vacuum and the residue is partitioned between ethyl acetate and water. The combined organic phases are dried and evaporated. The crude product is purified by silica gel flash chromatography.

With the choice of appropriate starting materials, which are mentioned herein or which can be obtained analogously or similarly to the described compounds, further relevant starting compounds, which afford final compounds of this invention, can be prepared analogously or similarly as described herein.

### A3. (E)-3-[1-(Pyridine-3-sulfonyl)-1H-pyrrol-3-yl]-N-(tetrahydro-pyran-2-yloxy)-acrylamide

The title compound can be prepared analogously to compound A1.

### A4. (E)-3-[1-(Benzothiazole-6-sulfanyl)-1H-pyrrol-3-yl]-N-(tetrahydro-pyran-2-yloxy)-acrylamide

To a solution of (E)-3-[1-Benzothiazole-6-sulfonyl)-1H-pyrrol-3-yl]-acrylic acid (240 mg) and N-hydroxybenzotriazole (HOBt) (110 mg) in DMF (10 ml) are added 1-ethyl-3-(3-dimethytaminopropyl)carbodiimide hydrochloride (EDC) (412 mg) and triethylamine (0.9 ml).

The mixture is stirred for 0.5 h at ambient temperature. To this solution is added o-(tetrahydro-pyran-2-yl)-hydroxylamine (84 mg). After completion of the reaction the mixture is evaporated at high vacuo and the product is extracted between ethylacetate and water. The organic phase is dried over sodium sulfate and the crude product is purified by silica gel flash chromatography. A nearly colorless oil (190 mg) in 60 % yield is obtained.

In a similar way can be prepared:
(E)-3-[1-(benzo[b]thiophene-2-sulfonyl)-1H-pyrrol-3-yl]-N-(tetrahydro-pyran-2-yloxy)-acrylamide,
(E)-3-[1-(1-Methyl-1H-imidazole-4-sulfonyl)-1H-pyrrol-3-yl]-N-(tetrahydro-pyran-2-yloxy)-acrylamide,
(2-{(E)-3-[1-(Benzo[b]thiophene-2-sulfonyl)-1H-pyrrol-3-yl]-allanoylamino}-phenyl)-carbamic acid tert-butyl ester,
(2-{(E)-3-[1-(Benzothiazole-6-sulfonyl)-1H-pyrrol-3-yl]-allanoylamino}-phenyl)-carbamic acid tert-butyl ester.

### A5. (E)-3-[1-(1-Methyl-1H-pyrazole-4-sulfonyl)-1H-pyrrol-3-yl]-N-(tetrahydro-pyran-2-yloxy)-acrylamide

153 mg NaH is suspended in 30 ml DMF. 300 mg (E)-3-(1H-pyrrol-3-yl)-N-(tetrahydro-pyran-2-yloxy)-acrylamide is added and the suspension is stirred 10 minutes at ambient temperature. 1-methyl-pyrazolidine-4-sufonyl chloride is added and the brown suspension is stirred at ambient temperature overnight The product is extracted between ethylacetate and water. The organic phase is dried over sodium sulfate and the crude product is purified by silica gel flash chromatography. By this method 79 mg of the title compound are obtained.

### B1. (E)-3-[1-(Naphthalene-2-sulfonyl)-1H-pyrrol-3-yl]-acrylic acid

A mixture of 1.044 g (E)-3-[1-(Naphthalene-2-sulfonyl)-1H-pyrrol-3-yl]-acrylic acid tert-butyl ester (compound C1) in 40 ml dichloromethane and 4 ml TFA is stirred at ambient temperature for 3 days. The reaction mixture is evaporated and the crude product is partitioned between dichloromethane and water. The organic phases are dried and evaporated. The title compound is obtained as nearly colorless solid.

### C1. (E)-3-[1-(Naphthalene-2-sulfonyl)-1H-pyrrol-3-yl]-acrylic acid tert-butyl ester

A mixture of 0.695g sodium hydride (60% in oil) and 50 ml dry THF is cooled to -30 °C. To this mixture are added (E)-3-(1H-Pyrrol-3-yl)-acrylic acid tert-butyl ester (compound D1). The suspension is stirred for 1 h at ambient temperature and cooled to -30°C. 5.62g naphthyl-2-sulfonylchloride are added and the mixture is stirred for 3 h at ambient temperature. After adding 10 ml water the mixture is extracted with ethyl acetate and the organic phase is dried and evaporated. The crude product is purified by means of silica gel chromatography.

### D1. (E)-3-(1H-Pyrrol-3-yl)-acrylic acid tert-butyl ester

5.29 g of sodium hydride 60% is supended in 100 ml of tetrahydrofurane under nitrogen at -30°C. 27.81 g of *tert*-butyl diphosphono acetate are added to the suspension and warmed slowly to room temperature and stirred for 30 minutes. Afterwards the mixture is recooled at -30°C and it is added 5.24 g of 1H-pyrrol-3-carbaldehyde (compound E1) and stirred at -30°C for 30 minutes. The suspension is warmed slowly to room temperature and 200 ml of aqueous ammonia solution are added. Then it is extracted with ethyl acetate. The combined organic phase is dried over Na₂SO₄, filtered and evaporated under vacuo. The crude product is purified by silica gel flash chromatography using a gradient of n-hexane-ethyl acetate from 2:1 to 1:1 to give 9.68 g of the title compound as a pale yellow solid.
MS (EI): 193.1 (M⁺); 137.1 (M⁺ -C₄H₈, 100%)
¹H-NMR (DMSO-d6): 1.45 (s, 9H); 5.96 (d, *J*= 15.7 Hz, 1H); 6.40 (m, 1H); 6.78 (m, 1H); 7.19 (m, 1H); 7.47 (d, *J*= 15.7 Hz, 1H); 11.11 (bs, exchangeable, 1H)

### E1. 1H-Pyrrol-3-carbaldehyde

4.70 g of dimethyl-(1H-pyrrol-3-ylmethylene)-ammonium chlorid (compound F1) are dissolved in 500 ml of 5.0% aqueous sodium hydroxide solution and stirred for 4 hours at ambient temperature. Afterwards the reaction mixture is extracted exhaustively with CH₂Cl₂. The combined organic phase is dried over Na₂SO₄. Then it is filtered and evaporated under vacuo. The crude product is purified by a silica gel flash chromatography using petroleum ether/diethylether 1:1 eluent to yield 3.01 g of the title compound as a pale yellow solid.
MS (EI):95.1 (M⁺, 100%)
¹H-NMR (DMSO-d6): 6.42 (dd, *J*₁ = 1.5 Hz, *J*₂= 6.5 Hz, 1H) ; 6.90 (m, 1H), 7.69 (dd, *J*₁ = 1.5 Hz, *J*₂= 6.4 Hz, 1H) ; 9.68 (s, 1H); 11.59 (bs, exchangeable, 1H)

### F1. Dimethyl-(1H-pyrrol-3-ylmethylene)-ammonium chlorid

10.60 g of (chloromethylene)dimethylammonium chloride and 6.25 g of N-(triisopropylsilyl)-pyrrole are suspended in 200 ml of CH₂Cl₂ under nitrogen at 0-5°C. The suspension is warmed to 60°C and stirred for 30 minutes. Afterwards the mixture is cooled to ambient temperature. The suspension is filtered and washed with diethylether to give 5.67 g of the title compound as grey solid.
MS (ESI): 123.3 (MH⁺, 100%)
¹H-NMR (DMSO-d6): 3.55 (s, 3H) ; 3.63 (s, 3H) ; 6.82 (m, *J*₁ =1.4 Hz, *J*₂ = 1.5Hz, *J*₃ = *J*₄ = 4.8 Hz, 1H); 7.22 (dd, *J*₁ = 4.7 Hz, *J*₂= 4.9, 1H), 8.00 (dd, *J*₁ = 1.6 Hz, *J*₂ =1.7 Hz, 1H) ; 8.78 (s, 1H) ; 12.94 (bs, exchangeable, 1H)

Using similar procedures to those described to attain to the abovementioned examples, but with suitable choice of the starting materials, which are described explicitly herein or which can be prepared in a manner known to the person skilled in the art or analogously or similarly to the materials described herein, further relevant compounds can be prepared.

### Commercial utility

The compounds according to this invention have valuable pharmacological properties and effects, which make them commercially applicable, such as e.g. they are commercially utilizable by properties related with inhibiting histone deacetylase activity and function.

"Histone deacetylase" (HDAC) means an enzyme with an activity towards the ε-acetyl group of lysine residues within a substrate protein. HDAC substrates are histone H2A, H2B, H3 or H4 proteins and isoforms but substrate proteins different to histones like, but not limited to, heat shock protein 90 (Hsp90), tubulin or the tumor suppressor protein p53 exist In particular histone deacetylases catalyse the hydrolysis the ε-acetyl group of lysine residues within these substrate proteins, forming the free amino group of lysine.

Inhibition of histone deacetylase by compounds according to this invention means inhibiting the activity and function of one or more HDAC isoenzymes, In particular isoenzymes selected from the so far known histone deacetylases, namely HDAC 1,2,3 and 8 (class I) and HDAC 4,5,6, 7, 10 (class II), HDAC 11 as well as the NAD+ dependent class III (Sir2 homologues). In some preferred embodiment this inhibition is at least about 50%, more preferable at least 75% and still more preferable above 90%. Preferably, this inhibition is specific to a specific histone deacetylase class (e.g HDAC class I enzymes), a selection of isoenzymes of highest pathophysiological relevance (e.g. HDAC 1,2,3 enzymes) or a single isoenzyme (e.g. the HDAC 1 enzyme). A histone deacetylase inhibitor in the meaning of this invention is therefore a compound capable of interacting with a histone deacetylase and inhibiting its activity, in particular its enzymatic activity. In this context "head group" defines the residues within a histone deacetylase inhibitor responsible for interacting with the active site of the enzyme, e.g the Zn²⁺ ion.

The inhibition of histone deacetylases is determined in biochemical assays of various formats and sources of enzymatic activity. HDAC activity is used either derived from nuclear or cellular extracts or by heterologous expression of defined HDAC isoenzymes in E.coli, insect cells or mammalian cells. Since HDAC isoenzymes are active in multiprotein complexes and form homo- and heterodimeres, nuclear extracts derived from human cancer cells, for example the human cervical carcinoma cell line HeLa, are preferred. These nuclear extracts contain class I and class II enzymes, but are enriched in class I enzymes. For expression of recombinant HDAC isoenzymes, mammalian expression systems like HEK293 cells are preferred. The HDAC isoenzyme is expressed as a fusion protein with an affinity tag, like the FLAG epitope. By affinity chromatography, the tagged protein is purified alone or in complex with endogenous proteins (e.g. other HDAC isoenzmyes and coactivators / platform proteins). The biochemical assays are well described and well known to persons skilled in the art As substrates, histone proteins, peptides derived from histone proteins or other HDAC substrates as well as acetylated lysine mimetics are used. One preferred promiscuous HDAC substrate is the tripeptide Ac-NH-GGK(Ac), coupled with the fluorophore 7-aminomethylcoumarin (AMC).

The invention further relates to the use of the compounds according to this invention for inhibiting histone deacetylase activity in cells and tissues, causing hyperacetylation of substrate proteins and as functional consequence, for example, the induction or repression of gene expression, induction of protein degradation, cell cycle arrest, induction of differentiation and/or induction of apoptosis.

The cellular activity of a histone deacetylase inhibitor includes any cellular effect related to histone deacetylase inhibition, in particular protein hyperacetylation, transcriptional repression and activation, induction of apoptosis, differentiation and / or cytotoxicity.

The term induction of apoptosis" and analogous terms are used to identity a compound which executes programmed cell death in cells contacted with that compound. "Apoptosis" is defined by complex biochemical events within the contacted cell, such as the activation of cysteine specific proteinases ("caspases") and the fragmentation of chromatin. Induction of apoptosis in cells contacted with the compound might not necessarily coupled with inhibition of cell proliferation or cell differentiation. Preferably, the inhibition of proliferation, induction of differentiation and/or induction of apoptosis is specific to cells with aberrant cell growth.
"induction of differentiation" Is defined as a process of cellular reprogramming leading to a reversible or irreversible cell cycle arrest in G0 and re-expression of a subset of genes typical for a certain specialized normal cell type or tissue (e.g. re-expression of milk fat proteins and fat in mammary carcinoma cells).
"Cytotoxicity" in general means arresting proliferation and/or inducing apoptotic cell death in vitro in mammalian cells, in particular human cancer cells.

Assays for quantification of cell proliferation, apoptosis or differentiation are well known to experts and state of the art For example, metabolic activity which is linked to cellular proliferation is quantified using the Alamar Blue / Resazurin assay (O'Brian et al. Eur j Biochem 267, 5421-U26, 2000) and induction of apoptosis is quantified by measurement of chromatin fragmentation with the cell death detection EUSA commercialized by Roche. Examples for cellular assays for the determination of hyperacetylation of HDAC substrates are given by measuring core histone acetylation using specific antibodies by Western blotting, reporter gene assays using respective responsive promoters or promoter elements (e.g. the p21 promotor or the sp1 site as responsive element) or finally by image analysis again using acetylation specific antibodies for core histone proteins.

Compounds according to this invention can be commercially applicable due to their HDAC inhibitory, anti-proliferative and/or apoptosis, inducing activity, which may be beneficial in the therapy or prophylaxis of diseases responsive thereto, such as e.g. any of those diseases mentioned herein.

The invention further relates to a method for treating, ameliorating or preventing cellular neoplasia by adminstration of an effective amount of a compound according to this invention to a mammal, in particular a human in need of such treatment. A "neoptasia" is defined by cells displaying aberrant cell proliferation and/or survival and/or a block in differentiation. The term "neoplasia" includes benign neoplasia, which is described by hyperproliferation of cells, incapable of forming an aggressive, metastasizing tumor in-vivo, and, in contrast, malignant neoplasia, which is described by cells with multiple cellular and biochemical abnormalities, capable of forming a systemic disease, for example forming tumor metastasis in distant organs.

Compounds according to this invention can be particularly used for the treatment of malignant neoplasia, also described as cancer, characterized by tumor cells finally metastasizing into distinct organs or tissues. Examples of malignant neoplasia treated with compounds according to the present invention include solid and haematological tumors. Solid tumors are exemplified by tumors of the breast, bladder, bone, brain, central and peripheral nervus system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva. Malignant neoplasia include inherited cancers exemplified by Retinoblastoma and Wilms tumor. In addition, malignant neoplasia include primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

It is to be noted that a cancer disease as well as a malignant neoplasia does not necessarily require the formation of metastases in distant organs. Certain tumors exert devastating effects on the primary organ itself through their aggressive growth properties. These can lead to the destruction of the tissue and organ structure finally resulting in failure of the assigned organ function.

Neoplastic cell proliferation might also effect normal cell behaviour and organ function. For example the formation of new blood vessels, a process described as neovascularization, is induced by tumors or tumor metastases. Compounds according to this invention can be commercially applicable for treatment of pathophysiological relevant processes caused by benign or neoplastic cell proliferation, such as but not limited to neovascularization by unphysiological proliferation of vascular endothelial cells.

Drug resistance is of particular importance for the frequent failure of standard cancer therapeutics. This drug resistance is caused by various cellular and molecular mechanisms like overexpression of drug efflux pumps, mutation within the cellular target protein or fusion proteins formed by chromosomal translocations. The commercial applicability of compounds according to the present invention is not limited to 1^{st} line treatment of patients. Patients with resistance to cancer chemotherapeutics or target specific anti-cancer drugs can be also amenable for treatment with these compounds for e.g. 2^{nd} or 3^{rd} line treatment cycles. A prominent example is given by acute promyelocytic leukemia patients with the PML-RARα fusion protein, resistant to standard therapy with retinoids. These patients can be resensitized towards retinoids by treatment with HDAC inhibitory drugs like the compounds according to the present invention.

The invention further provides to a method for treating a mammal, in particular a human, bearing a disease different to cellular neoplasia, sensitive to histone deacetylase inhibitor therapy comprising administering to said mammal a pharmacologically active and therapeutically effective and tolerable amount of a compound according to this invention. These non malignant diseases include
(i) arthropathies and osteopathological diseases such as rheumatoid arthritis, osteoarthrtis, gout, polyarthritis and psoriatic arthritis
(ii) autoimmune diseases like systemic lupus erythematosus and transplant rejection
(iii) hyperproliferative diseases such as psoriasis or smooth muscle cell proliferation including vascular proliferative disorders, atherosclerosis and restenosis
(iv) acute and chronic inflammatory diseases and dermal diseases such as ulcerative colitis, Crohn's disease, allergic rhinitis, allergic dermatitis, cystic fibrosis, chronic obstructive bronchitis and asthma
(v) endometriosis, uterine fibroids, endometrial hyperplasia and benign prostate hyperplasia
(vi) cardiac dysfunction
(vii) inhibiting immunosuppressive conditions like HIV infections
(viii) neuropathological disorders like Parkinson disease, Alzheimer disease or polyglutamine related disorders
(ix) pathological conditions amenable to treatment by potentiating of endogenous gene expression as well as enhancing transgene expression in gene therapy.

Compounds according to the present invention may commercially applicable for treatment, prevention or amelioration of the diseases of benign and malignant behavior as described herein, such as, for example, (hyper)proliferative diseases and/or disorders responsive to induction of apoptosis and/or disorders responsive to cell differentiation, e.g. benign or malignant neoplasia, particularly cancer, such as e.g. any of those cancer diseases described above.

In the context of their properties, functions and usabilities mentioned herein, the compounds according to the present invention are expected to be distinguished by valuable and desirable effects related therewith, such as e.g. by low toxicity, superior bioavailability in general (such as e.g. good enteral absorption), superior therapeutic window, absence of significant side effects, and/or further beneficial effects related with their therapeutic and pharmaceutical suitability.

The present invention further includes a method for the treatment of mammals, including humans, which are suffering from one of the abovementioned conditions, illnesses, disorders or diseases. The method comprises that a pharmacologically active and therapeutically effective and tolerable amount of one or more of the compounds according to this invention, which function by inhibiting histone deacetylases and -in general- by modulating protein acetylation, induce various cellular effects, in particular induction or repression of gene expression, arresting cell proliferation, inducing cell differentiation and/or inducing apoptosis, is administered to the subject in need of such treatment.

The invention further includes a method for treating diseases and/or disorders responsive or sensitive to the inhibition of histone deacetylases, particularly those diseases mentioned above, such as e.g. cellular neoplasia or diseases different to cellular neoplasia as indicated above, in mammals, including humans, suffering therefrom comprising administering to said mammals in need thereof a pharmacologically active and therapeutically effective and tolerable amount of one or more of the compounds according to the present invention.

The present invention further includes a therapeutic method useful to modulate protein acetylation, gene expression, cell proliferation, cell differentiation and/or apoptosis in vivo in diseases mentioned above, in particular cancer, comprising administering to a subject in need of such therapy a pharmacologically active and therapeutically effective and tolerable amount of one or more of the compounds according to this invention, which function by inhibiting histone deacetylases.

The present invention further provides a method for regulating endogenous or heterologous promotor activity by contacting a cell with a compound according to this invention.

The invention further relates to the use of the compounds according to the present invention for the production of pharmaceutical compositions which are employed for the treatment and/or prophylaxis and/or amelioration of the diseases, disorders, illnesses and/or conditions as mentioned herein.

The invention further relates to the use of the compounds according to the present invention for the production of pharmaceutical compositions which are employed for the treatment and/or prophylaxis of diseases and/or disorders responsive or sensitive to the inhibition of histone deacetylases, particularly those diseases mentioned above, such as e.g. cellular neoplasia or diseases different to cellular neoplasia as indicated above.

The invention further relates to the use of the compounds according to the present invention for the production of pharmaceutical compositions having histone deacetylases inhibitory activity.

The invention further relates to the use of the compounds according to the present invention for the production of pharmaceutical compositions for inhibiting or treating cellular neoplasia, such as benign or malignant neoplasia, e.g. cancer.

The invention further relates to the use of the compounds according to the present invention for the production of pharmaceutical compositions which can be used for treating, preventing or ameliorating of diseases responsive to arresting aberrant cell growth, such as e.g. (hyper)proliferative diseases of benign or malignant behaviour, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of disorders responsive to induction of apoptosis, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of disorders responsive to induction of differentiation, such as e.g. any of those diseases mentioned herein, particularly cancer, such as e.g. any of those cancer diseases described herein above.

The invention further relates to the use of the compounds according to the present invention for the production of pharmaceutical compositions which can be used for treating, preventing or ameliorating of benign or malignant neoplasia, particularly cancer, such as e.g. any of those cancer diseases described herein above.

The invention further relates to the use of the compounds according to the present invention for the production of pharmaceutical compositions for the treatment of a disease different to a cellular neoplasia and sensitive to histone deacetylase inhibitor therapy, such as the non-malignant diseases mentioned before.

The invention further relates to the use of the compounds according to the present invention for the production of pharmaceutical compositions for inhibiting histone deacetylase activity in the treatment of diseases responsive to said inhibition or to the functional consequences thereof.

The invention further relates to a method for treating, preventing or ameliorating the diseases, disorders, illnesses and/or conditions mentioned herein in a mammal, in particular a human patient, comprising administering a pharmacologically active and therapeutically effective and tolerable amount of one or more compounds according to the present invention to said mammal in need thereof.

The invention further relates to the compounds according to this invention for use in the treatment and/or prophylaxis of diseases, especially the diseases mentioned.

The invention further relates to pharmaceutical compositions comprising one or more of the compounds according to this invention and a pharmaceutically acceptable carrier or diluent.

The present invention further relates to pharmaceutical compositions comprising one or more of the compounds according to this invention and pharmaceutically acceptable auxiliaries and/or excipients.

The invention further relates to a combination comprising one or more of the compounds according to this invention and a pharmaceutically acceptable diluent, excipient and/or carrier, e.g. for treating, preventing or ameliorating (hyper)proliferative diseases of benign or malignant behaviour and/or disorders responsive to induction of apoptosis, such as, for example, benign or malignant neoplasia, e.g. cancer, such as e.g. any of those cancer diseases described herein above.

The invention further relates to pharmaceutical compositions according to this invention having histone deacetylases inhibitory activity.

The invention further relates to pharmaceutical compositions according to this invention having apoptosis inducing activity.

The invention further relates to pharmaceutical compositions according to this invention having anti-proliferative activity.

The invention further relates to pharmaceutical compositions according to this invention having cell differentiation inducing activity.

The invention further relates to the use of a pharmaceutical composition comprising one or more of the compounds according to this invention and a pharmaceutically acceptable carrier or diluent in the manufacture of a pharmaceutical product, such as e.g. a commercial package, for use in the treatment and/or prophylaxis of the diseases as mentioned.

Additionally, the invention relates to an article of manufacture, which comprises packaging material and a pharmaceutical agent contained within said packaging material, wherein the pharmaceutical agent is therapeutically effective for inhibiting the effects of histone deacetylases, ameliorating the symptoms of an histone deacetylase mediated disorder, and wherein the packaging material comprises a label or package insert which indicates that the pharmaceutical agent is useful for preventing or treating histone deacetylase mediated disorders, and wherein said pharmaceutical agent comprises one or more compounds of formula I according to the invention. The packaging material, label and package insert otherwise parallel or resemble what is generally regarded as standard packaging material, labels and package inserts for pharmaceuticals having related utilities.

The pharmaceutical compositions according to this invention are prepared by processes which are known per se and familiar to the person skilled in the art. As pharmaceutical compositions, the compounds of the invention (= active compounds) are either employed as such, or preferably in combination with suitable pharmaceutical auxiliaries and/or excipients, e.g. in the form of tablets, coated tablets, capsules, caplets, suppositories, patches (e.g. as TTS), emulsions, suspensions, gels or solutions, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, vehicles, excipients, diluents, carriers or adjuvants which are suitable for the desired pharmaceutical formulations, preparations or compositions on account of his/her expert knowledge. In addition to solvents, gel formers, ointment bases and other active compound excipients, for example antioxidants, dispersants, emulsifiers, preservatives, solubilizers, colorants, complexing agents or permeation promoters, can be used.

The administration of the compounds, pharmaceutical compositions or combinations according to the invention may be performed in any of the generally accepted modes of administration available in the art. Illustrative examples of suitable modes of administration include intravenous, oral, nasal, parenteral, topical, transdermal and rectal delivery. Oral and intravenous delivery are preferred.

For the treatment of dermatoses, compounds according to this invention are in particular administered in the form of those pharmaceutical compositions which are suitable for topical application. For the production of the pharmaceutical compositions, the compounds of the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The pharmaceutical compositions according to the invention are prepared by processes known per se. The dosage of the compounds of the invention (= active compounds) is carried out in the order of magnitude customary for histone deacetylases inhibitors. Topical application forms (such as ointments) for the treatment of dermatoses thus contain the active compounds in a concentration of, for example, 0.1-99%. The customary dose in the case of systemic therapy (p.o.) may be between 0.03 and 60 mg/kg per day, (i. v.) may be between 0.03 and 60 mg/kg/h. In another embodiment, the customary dose in the case of systemic therapy (p.o.) is between 0.3 and 30 mg/kg per day, (i. v.) is between 0.3 and 30 mg/kg/h.

The choice of the optimal dosage regime and duration of medication, particularly the optimal dose and manner of administration of the active compounds necessary in each case can be determined by a person skilled in the art on the basis of his/her expert knowledge.

Depending upon the particular disease, to be treated or prevented, additional therapeutic active agents, which are normally administered to treat or prevent that disease, may optionally be coadministered with the compounds according to the present invention. As used herein, additional therapeutic agents that are normally administered to treat or prevent a particular disease are known as appropriate for the disease being treated.

For example, compounds according to this invention may be combined with one or more standard therapeutic agents or radiation used for treatment of the diseases as mentioned before.

Thus, in one particular embodiment compounds according to this invention may be combinded with one or more art-known anti-cancer agents, such as e.g. with one or more art-known chemotherapeutic and/or target specific anti-cancer agents as described below, and/or radiation.

Examples of know chemotherapeutic anti-cancer agents frequently used in combination therapy include, but are not limited to (i) alkylabng/carbamylating agents such as Cyclophosphamid (Endoxan®), Ifosfamid (Holoxan®), Thiotepa (Thiotepa Lederle®), Melphalan (Alkeran®), or chloroethylnitrosourea (BCNU); (ii) platinum derivatives like cls-platin (Platinex® BMS), oxaliplatin or carboplatin (Cabroplat® BMS); (iii) antimitotic agents / tubulin inhibitors such as vinca alkaloids (vincristine, vinblastine, vinorelbine), taxanes such as Paclitaxel (Taxol®), Docetaxel (Taxotere®) and analogs as well as new formulations and conjugates thereof, epothilones such as Epothilone B (Patupilane®), Azaepothilone (Ixabepilone®) or ZK-EPO, a fully synthetic epothilone B analogs; (iv) topoisomerase inhibitors such as anthracyclines (exemplified by Doxorubicin / Adriblastin®), epipodaphyllotoxines (exemplified by Etoposide / Etopophos®) and camptothecin and camptothecin analogs (exemplified by Irinotecan / Camptosar® or Topotecan / Hycamtin®); (v) pyrimidine antagonists such as 5-fluorouracil) (5-FU), Capecitabine (Xeloda®), Arabinosylcytosine / Cytarabin (Atexen®) or Gemcitabine (Gemzar®); (vi) purin antagonists such as 6-mercaptopurine (Puri-Nethol®), 6-thloguanine or Hudarabine (Fludara®) and finally (vii) folic acid antagonists such as methotrexate (Farmitrexat®) or premetrexed (Alimta®).

Examples of target specific anti-cancer drug classes used in experimental or standard cancer therapy include but are not limited to (i) kinase inhibitors such as e.g. imatinib (Glivec®), ZD-1839 / Gefitinib (Iressa®), Bay43-9006 (Sorafenib), SU112481 / Sunitinib (Sutent®) or OSI-774 / Erlotinib (Tarceva®); (li) proteasome Inhibitors such as PS-341 / Bortezumib (Velcade®); (iii) heat shock protein 90 inhibitors like 17-allylaminogeldanamycin (17-AAG); (iv) vascular targeting agents (VTAs) like combretastin A4 phosphate or AVE8062 / AC7700 and anti-angiogenic drugs like the VEGF antibodies, such as Bevacizumab (Avastin®), or KDR tyrosine kinase inhibitors such as PTK787 / ZK222584 (Vatalanib); (v) monoclonal antibodies such as Trastuzumab (Herceptin®) or Rituximab (MabThera / Rituxan®) or Alemtuzumab (Campath®) or Tositumab (Sexxar®) or C225/ Cetuximab (Erbitux®) or Avastin (see above) as well as mutants and conjugates of monoclonal antibodies, e.g. Gemtuzumab ozogamicin (Mylotarg®) or Ibritumomab tiuxetan (Zevatin®), and antibody fragments; as well as mutants and conjugates of monoclonal antibodies and antibody fragments; (vi oligonucleotide based therapeutics like G-3139 / Oblimersen (Genasense®); (vii) Tol-like receptor / TLR 9 agonists like Promune®, TLR 7 agonists like Imiquimod (Aldara®) or Isatoribine and analogues thereof, or TLR 7/8 agonists like Resiquimod as well as immunostimulatory RNA as TLR 7/8 agonists; (viii) protease inhibitors (ix) hormonal therapeutics such as anti-estrogens (e.g. Tamoxifen or Raloxifen), anti-androgens (e.g. Flutamide or Casodex), LHRH analogs (e.g. Leuprolide, Goserelin or Triptorelin) and aromatase inhibitors.

Other known target specific anti-cancer agents which can be used for combination therapy include bleomycin, retinoids such as all-trans retinoic acid (ATRA), DNA methyltransferase inhibitors such as the 2-deoxycytidine derivative Decitabine (Docagen®) and 5-Azacytidine, alanosine, cytokines such as interleukin-2, interferons such as interferon α2 or interferon-γ, death receptor agonists, such as TRAIL, DR4/5 agonistic antibodies, FasL and TNF-R agonists, and finally histone deacetylase inhibitors different to the compounds according to this invention such as SAHA, PXD101, MS275, MGCD0103, Depsipeptide / FK228, NVP-LBH589, NVP-LAQ824, Valproic acid (VPA) and butyrates.

As exemplary anti-cancer agents for use in combination with the compounds according to this invention in the cotherapies mentioned herein any of the following drugs may be mentioned, without being restricted thereto, 5 FU, actinomycin D, ABARELIX, ABCIXIMAB, ACLARUBICIN, ADAPALENE, ALEMTUZUMAB, ALTRETAMINE, AMINOGLUTETHIMIDE, AMIPRILOSE, AMRUBICIN, ANASTROZOLE, ANCITABINE, ARTEMISININ, AZATHIOPRINE, BASILIXIMAB, BENDAMUSTINE, BEVACIZUMAB, BEXXAR, BICALUTAMIDE, BLEOMYCIN, BORTEZOMIB, BROXURIDINE, BUSULFAN, CAMPATH, CAPECITABINE, CARBOPLATIN, CARBOQUONE, CARMUSTINE, CETRORELIX, CHLORAMBUCIL, CHLORMETHINE, CISPLATIN, CLADRIBINE, CLOMIFENE, CYCLOPHOSPHAMIDE, DACARBAZINE, DACLIZUMAB, DACTINOMYCIN, DAUNORUBICIN, DECITABINE, DESLORELIN, DEXRAZOXANE, DOCETAXEL, DOXIFLURIDINE, DOXORUBICIN, DROLOXIFENE, DROSTANOLONE, EDELFOSINE, EFLORNITHINE, EMITEFUR, EPIRUBICIN, EPITIOSTANOL, EPTAPLATIN, ERBITUX, ERLOTINIB, ESTRAMUSTINE, ETOPOSIDE, EXEMESTANE, FADROZOLE, FINASTERIDE, FLOXURIDINE, FLUCYTOSINE, FLUDARABINE, FLUOROURACIL, FLUTAMIDE, FORMESTANE, FOSCARNET, FOSFESTROL, FOTEMUSTINE, FULVESTRANT, GEFITINIB, GENASENSE, GEMCITABINE, GLIVEC, GOSERELIN, GUSPERIMUS, HERCEPTIN, IDARUBICIN, IDOXURIDINE, IFOSFAMIDE, IMATINIB, IMPROSULFAN, INFLIXIMAB, IRINOTECAN, IXABEPILONE, LANREOTIDE, LETROZOLE, LEUPRORELIN, LOBAPLATIN, LOMUSTINE, LUPROLIDE, MELPHALAN, MERCAPTOPURINE, METHOTREXATE, METUREDEPA, MIBOPLATIN, MIFEPRISTONE, MILTEFOSINE, MIRIMOSTIM, MITOGUAZONE, MITOLACTOL, MITOMYCIN, MITOXANTRONE, MIZORIBINE, MOTEXAFIN, MYLOTARG, NARTOGRASTIM, NEBAZUMAB, NEDAPLATIN, NILUTAMIDE, NIMUSTINE, OCTREOTIDE, ORMELOXIFENE, OXALIPLATIN, PACLITAXEL, PALIVIZUMAB, PATUPILONE, PEGASPARGASE, PEGFILGRASTIM, PEMETREXED, PENTETREOTIDE, PENTOSTATIN, PERFOSFAMIDE, PIPOSULFAN, PIRARUBICIN, PLICAMYCIN, PREDNIMUSTINE, PROCARBAZINE, PROPAGERMANIUM, PROSPIDIUM CHLORIDE, RALOXIFEN, RALTITREXED, RANIMUSTINE, RANPIRNASE, RASBURICASE, RAZOXANE, RITUXIMAB, RIFAMPICIN, RITROSULFAN, ROMURTIDE, RUBOXISTAURIN, SARGRAMOSTIM, SATRAPLATIN, SIROLIMUS, SOBUZOXANE, SORAFENIB, SPIROMUSTINE, STREPTOZOCIN, SUNITINIB, TAMOXIFEN, TASONERMIN, TEGAFUR, TEMOPORFIN, TEMOZOLOMIDE, TENIPOSIDE, TESTOLACTONE, THIOTEPA, THYMALFASIN, TIAMIPRINE, TOPOTECAN, TOREMIFENE, TRAIL, TRASTUZUMAB, TREOSULFAN, TRIAZIQUONE, TRIMETREXATE, TRIPTORELIN, TROFOSFAMIDE, UREDEPA, VALRUBICIN, VATALANIB, VERTEPORFIN, VINBLASTINE, VINCRISTINE, VINDESINE, VINORELBINE, VOROZOLE and ZEVALIN.

The anti-cancer agents mentioned herein above as combination partners of the compounds according to this invention are meant to include pharmaceutically acceptable derivatives thereof, such as e.g. their pharmaceutically acceptable salts.

The person skilled in the art is aware on the base of his/her expert knowledge of the kind, total daily dosage(s) and administration form(s) of the additional therapeutic agent(s) coadministered. Said total daily dosage(s) can vary within a wide range.

In practicing the present invention and depending on the details, characteristics or purposes of their uses mentioned above, the compounds according to the present invention may be administered in combination therapy separately, sequentially, simultaneously, concurrently or chronologically staggered (such as e.g. as combined unit dosage forms, as separate unit dosage forms, as adjacent discrete unit dosage forms, as fixed or non-fixed combinations, as kit-of-parts or as admixtures) with one or more standard therapeutics, in particular, art-known anti-cancer agents (chemotherapeutic and/or target specific anti-cancer agents, such as e.g. any of those mentioned above.

In this context, the present invention further relates to a combination comprising
a first active ingredient, which is at least one compound according to this invention, and a second active ingredient, which is at least one art-known standard therapeutic, for example an art-known anti-cancer agent, such as e.g. one or more of those mentioned herein above,
for separate, sequential, simultaneous, concurrent or chronologically staggered use in therapy, such as e.g. in therapy of any of those diseases mentioned herein.

The term "combination" according to this invention may be present as a fixed combination, a non-fixed combination or a kit-of-parts.

A "fixed combination" is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A "kit-of-parts" is defined as a combination wherein the said first active ingredient and the said second active ingredient are present in more than one unit. One example of a "kit-of-parts" is a combination wherein the said first active ingredient and the said second active ingredient are present separately. The components of the kit-of-parts may be administered separately, sequentially, simultaneously, concurrently or chronologically staggered.

The present invention further relates to a pharmaceutical composition comprising
a first active ingredient, which is at least one compound according to this invention, and a second active ingredient, which is at least one art-known anti-cancer agent, such as e.g. one or more of those mentioned herein above, and, optionally,
a pharmaceutically acceptable carrier or diluent,
for separate, sequential, simultaneous, concurrent or chronologically staggered use in therapy, such as e.g. in therapy of diseases responsive or sensitive to the inhibition of histone deacetylases, particularly (hyper)proliferative diseases and/or disorders responsive to induction of apoptosis, such as e.g. any of those diseases mentioned herein, like benign or malignant neoplasia, especially cancer, particularly any of those cancer diseases described above.

The present invention further relates to a combination product comprising
a.) at least one compound according to this invention formulated with a pharmaceutically acceptable carrier or diluent, and
b.) at least one art-known anti-cancer agent, such as e.g. one or more of those mentioned herein above, formulated with a pharmaceutically acceptable carrier or diluent.

The present invention further relates to a kit-of-parts comprising a preparation of a first active ingredient, which is a compound according to this invention, and
a pharmaceutically acceptable carrier or diluent; a preparation of a second active ingredient, which is an art-known anti-cancer agent, such as one of those mentioned above, and a pharmaceutically acceptable carrier or diluent; for simultaneous, concurrent, sequential, separate or chronologically staggered use in therapy. Optionally, said kit comprises instructions for its use in therapy, e.g. to treat diseases responsive or sensitive to the inhibition of histone deacetylases, such as e.g. cellular neoplasia or diseases different to cellular neoplasia as indicated above, particularly cancer, such as e.g. any of those cancer diseases described above.

The present invention further relates to a combined preparation comprising at least one compound according to this invention and at least one art-known anti-cancer agent for simultaneous, concurrent, sequential or separate administration.

In this connection, the present invention further relates to combinations, compositions, formulations, preparations or kits according to the present invention having histone deacetylases inhibitory activity.

Also in this connection, the present invention further relates to combinations, compositions, formulations, preparation or kits according to the present invention having anti-(hyper)proliferative and/or apoptosis inducing activity.

In addition, the present invention further relates to a method for treating in combination therapy diseases responsive or sensitive to the inhibition of histone deacetylases, such as e.g. those mentioned above, e.g. (hyper)proliferative diseases and/or disorders responsive to induction of apoptosis, like cancer, in a patient comprising administering a combination, composition, formulation, preparation or kit as described herein to said patient in need thereof.

In addition, the present invention further relates to a method for treating diseases responsive or sensitive to the inhibition of histone deacetylases, such as e.g. cancer, in a patient comprising administering in combination therapy separately, simultaneously, concurrently, sequentially or chronologically staggered a pharmaceutically active and therapeutically effective and tolerable amount of a pharmaceutical composition, which comprises a compound according to this invention and a pharmaceutically acceptable carrier or diluent, and a pharmaceutically active and therapeutically effective and tolerable amount of one or more art-known anti-cancer agents, such as e.g. one or more of those mentioned herein, to said patient in need thereof.

In further addition, the present invention relates to a method for treating, preventing or ameliorating (hyper)proliferative diseases and/or disorders responsive to induction of apoptosis, such as e.g. benign or malignant neoplasia, e.g. cancer, particularly any of those cancer diseases mentioned herein, in a patient comprising administering separately, simultaneously, concurrently, sequentially or chronologically staggered to said patient in need thereof an amount of a first active compound, which is a compound according to the present invention, and an amount of at least one second active compound, said at least one second active compound being a standard therapeutic agent, particularly at least one art-known anti-cancer agent, such as e.g. one or more of those chemotherapeutic and target-specific anti-cancer agents mentioned herein, wherein the amounts of the first active compound and said second active compound result in a therapeutic effect

In yet further addition, the present invention relates to a method for treating, preventing or ameliorating (hyper)proliferative diseases and/or disorders responsive to induction of apoptosis, such as e.g. benign or malignant neoplasia, e.g. cancer, particularly any of those cancer diseases mentioned herein, in a patient comprising administering a combination according to the present invention.

In addition, the present invention further relates to the use of a composition, combination, formulation, preparation or kit according to this invention in the manufacture of a pharmaceutical product, such as e.g. a commercial package or a medicament, for treating, preventing, or ameliorating diseases responsive or sensitive to the inhibition of histone deacetylases, particularly those diseases mentioned herein, such as e.g. benign or malignant neoplasia, particularly cancer.

The present invention further relates to a commercial package comprising one or more compounds of the present invention together with instructions for simultaneous, concurrent, sequential or separate use with one or more chemotherapeutic and/or target specific anti-cancer agents, such as e.g. any of those mentioned herein.

The present invention further relates to a commercial package consisting essentially of one or more compounds of the present invention as sole active ingredient together with instructions for simultaneous, concurrent, sequential or separate use with one or more chemotherapeutic and/or target specific anti-cancer agents, such as e.g. any of those mentioned herein.

The present invention further relates to a commercial package comprising one or more chemotherapeutic and/or target specific anti-cancer agents, such as e.g. any of those mentioned herein, together with instructions for simultaneous, concurrent, sequential or separate use with one or more compounds according to the present invention.

The compositions, combinations, preparations, formulations, kits or packages mentioned in the context of the combination therapy according to this invention may also include more than one of the compounds according to this invention and/or more than one of the art-known anti-cancer agents mentioned.

The first and second active ingredient of a combination or kit-of-parts according to this invention may be provided as separate formulations (i.e. independently of one another), which are subsequently brought together for simultaneous, sequential, separate or chronologically staggered use in combination therapy; or packaged and presented together as separate components of a combination pack for simultaneous, concurrent, sequential, separate or chronologically staggered use in combination therapy.

The type of pharmaceutical formulation of the first and second active ingredient of a combination or kit-of-parts according to this invention can be similar, i.e. both ingredients are formulated in separate tablets or capsules, or can be different, i.e. suited for different administration forms, such as e.g. one active ingredient is formulated as tablet or capsule and the other is formulated for e.g. intravenous administration.

The amounts of the first and second active ingredients of the combinations, compositions or kits according to this invention may together comprise a therapeutically effective amount for the treatment, prophylaxis or amelioration of a disease responsive or sensitive the inhibition of histone deacetylases, such as, for example, one of those diseases mentioned herein, e.g. benign or malignant neoplasia, particularly cancer, like any one of those cancer diseases mentioned herein.

In addition, compounds according to the present invention can be used in the pre- or post-surgical treatment of cancer.

In further addition, compounds according to the present invention can be used in combination with radiation therapy, in particular in sensitization of cancer patients towards standard radiation therapy.

A combination according to this invention can refer to a composition comprising both the compound(s) according to this invention and the other active anti-cancer agent(s) in a fixed combination (fixed unit dosage form), or a medicament pack comprising the two or more active ingredients as discrete separate dosage forms (non-fixed combination). In case of a medicament pack comprising the two or more active ingredients, the active ingredients are preferably packed into blister cards which are suited for improving compliance.

Each blister card preferably contains the medicaments to be taken on one day of treatment. If the medicaments are to be taken at different times of day, the medicaments can be disposed in different sections on the blister card according to the different ranges of times of day at which the medicaments are to be taken (for example morning and evening or morning, midday and evening). The blister cavities for the medicaments to be taken together at a particular time of day are accommodated in the respective range of times of day. The various times of day are, of course, also put on the blister in a clearly visible way. It is also possible, of course, for example to indicate a period in which the medicaments are to be taken, for example stating the times.

The daily sections may represent one line of the blister card, and the times of day are then identified in chronological sequence in this column.

Medicaments which must be taken together at a particular time of day are placed together at the appropriate time on the blister card, preferably a narrow distance apart, allowing them to be pushed out of the blister easily, and having the effect that removal of the dosage form from the blister is not forgotten.

### Biological Investigations

### Isolation of HDAC activity from HeLa cell nuclei:

HDAC activity is isolated from nuclear HeLa extracts according to a method originally described by Dignam et al. (Nucl. Acids Res. 11, pp1475, 1983). Briefly, nuclei isolated from HeLa cells (CIL SA, Seneffe, Belgium) are resuspended in buffer C (20mM Hepes pH 7.9, 25% v:v glycerol, 0.42M NaCl, 1.5mM MgCl₂, 0.2mM EDTA, 0.5mM PefaBloc and 0.5mM DTT) and stirred for 30min on ice. After centrifugation, the supernatant is dialysed against buffer D (40mM Tris HCl pH 7.4, 100mM KCI, 0.2mM EDTA, 0.5mM DTT and 28% v:v glycerol) for 5h at 4°C. After dialysis and centrifugation, the supernatant is stored in aliquots at -80°C and used for Western blot analysis as well as the enzymatic assay as described in the following.

### Isolation of rHDAC1

Human HDAC1 fused with the flag epitope is stably expressed in Hek293 cells. After mass cultivation in DMEM with supplements and 2% fetal calf serum, cells are lysed and flag-HDAC1 purified by M2-agarose affinity chromatography as described (Sigma Art No. A-2220). Fractions from the purification are analysed by Western bloting as well as tested for enzymatic activity as described below.

### Fluorimetric HDAC activity assay:

The HDAC enzyme activity assay is done as described by Wegener et al. (Chem. & Biol. 10, 61-68, 2003). Briefly 40µl of a 1:100 dilution (= 0.4µl) nuclear HeLa extract (mixture of class 1 and II HDACs), 29 µl enzyme buffer (15mM Tris HCl pH 8.1, 0.26mM EDTA, 250mM NaCl, 10% v:v glycerol) and 10µl test compound are added to a well of a 96well microtiter plate and reaction started by addition of 30µl substrate (Ac-NH-GGK(Ac)-AMC; final concentration 25µM and final volume 100µl). After incubation for 90min at 30°C, reaction is terminated by the addition of 25µl stop solution (50mM Tris HCl pH 8, 100mM NaCl, 0.5mg/ml trypsine and 2µM TSA). After incubation at room temperature for further 40min, fluorescence is measured using a Wallac Victor 1420 multilabel counter (Ex 355nm, Em 480nm) for quantification of AMC (7-amino-4-methytcoumarin) generated by trypsine cleavage of the deacetylated peptide. For the calculation of IC₅₀ values the fluorescence in wells without test compound (1% DMSO, negative control) is set as 100% enzymatic activity and the fluorescence in wells with 2µM TSA (positive control) are set at 0% enzymatic activity. The corresponding IC₅₀ values of the compounds for HDAC inhibitory activity are determined from the concentration-effect curves by means of non-linear regression.

The HDAC1 enzymatic assay is done with slight modifications with recombinant flag-HDAC1 protein isolated from HEK293 cell lysates. About 10ng/well flag-HDAC1 are incubated with 6µM Ac-NH-GGK(Ac)-AMC substrate for 3h at 30°C. Termination of the reaction and all further steps are done as described for HeLa cell nuclear extracts as a source for HDAC enzymatic activity.

HDAC activity derived from HeLa cell nuclear extracts is inhibited by Examples 1 to 4, and 7 to 12 with an IC₅₀ values in the range of 2.5µM to 0.0039µM. Recombinant human HDAC1 expressed in Hek293 cells is inhibited by Examples 1 to 3, and 7 to 12 with IC₅₀ values in the range of 1µM to 0.0039µM.

### Cellular Histone H3 hyperacetylation assay:

To assess the cellular efficacy of a histone deacetylase inhibitor in vitro, an assay is set up in black clear-bottom 96-well plates and optimized for use on the Cellomics "ArrayScan II" platform for a quantitative calculation of histone acetylation. The protocol uses a polyclonal rabbit antibody, specifically binding to acetylated lysine 23 or, alternatively, acetylated lysine 9 + 14 of human histone H3 on fixed cells with an Alexa Fluor 488 labeled goat anti rabbit-IgG used for counterstaining (modified from Braunger et al. AACR annual conference 2003, Abstract 4556).

5x10³ HeLa cervical carcinoma cells/well (ATCC CCL-2) in 200µl Dulbecco's modified Eagle's medium (DMEM) containing 10% fetal calf serum are seeded at day 1 in Packard view plates and incubated for 24h under standard cell culture conditions. On day 2,1µl test compound (200x final concentration) is added and incubation continued for further 24h. On day 3, the culture medium is discarded and attached cells fixed for 15min at room temperature by addition of 100µl fixation buffer (3.7% v:v formaldehyde in phosphate buffered saline / PBS). After discarding the fixation buffer and one wash with blocking solution (1% BSA, 0,3% Tween 20 in PBS), cells are permeabilized at room temperature by addition of 100µl/well permeabilization buffer (30,8 mM NaCl, 0,54 mM Na₂HPO₄, 0,31 mM KH₂PO₄, 0,1% v:v Triton X-100) for 15min at room temperature. After discarding the permeabilization buffer and washing twice with 100µl/well blocking solution at room temperature, the 1^{st} antibody (anti-K23 histone H3 antibody, Cell Signaling No. 9674 or, alternatively, anti-K9+14 histone H3 antibody, Calbiochem No. 382158) in blocking solution (50µl/well) is added. After incubation for 1 h at room temperature, the wells are washed twice at room temperature with 100µl/ well blocking solution before additon of the 2^{nd} antibody (goat-anti-rabbit Alexa Fluor 488; MoBTec No. A-11008) in blocking solution (50µl/ well). After further incubation for 1 h at room temperature, wells are washed twice with 100µl/ well blocking solution at room temperature. Finally, 100µl/well PBS are added and image analysis performed on the Cellomics "ArrayScan II" platform. For EC₅₀ determination, the percentage of positive cells showing nuclear fluorescence is determined and EC₅₀ calculation done from concentration-effect curves by means of non-linear regression. For calibration, a positive (reference HDAC inhibitors like SAHA or LBH589) and negative control is included.

Cellular histone hyperacetylation in HeLa cells is induced by Examples 1, 2, 4, 9 and 10 in the range of EC₅ = 10µM to 0.19µM.

### Cellular cytotoxicity assay:

The anti-proliferative activity of the histone deacetylase inhibitory compounds as described herein, is evaluated with the HeLa cervical carcinoma cell line (ATCC CCL2) and A549 non-small cell lung cancer cell line (ATCC CCL185) using the Alamar Blue (Resazurin) cell viability assay (O'Brien et al. Eur J Biochem 267, 5421-5426, 2000). Resazurin is reduced to the fluorescent resorufin by cellular dehydrogenase activity, correlating with viable, proliferating cells. Test compounds are dissolved as 20 mM solutions in dimethylsulfoxide (DMSO) and subsequently diluted in semi-logarithmic steps. HeLa or A549 cells are seeded into 96 well flat bottom plates at a density of 1000 and 2000 cells per well, respectively, in a volume of 200 µl per well. 24 hours after seeding 1 µl each of the compound dilutions are added into each well of the 96 Well plate. Each compound dilution is tested as quadruplicates. Wells containing untreated control cells are filled with 200 µl DMEM medium containing 0.5% v:v DMSO. The cells are then incubated with the substances for 48 or 72 hours at 37°C in a humidified atmosphere containing 5% carbon dioxide. To determine the viability of the cells, 20 µl of an Resazurin solution (Sigma; 90mg /l) were added. After4 hours incubation at 37°C the fluorescence Is measured at an extinction of 544 nm and an emission of 590 nm. For the calculation of the cell viability the emission value from untreated cells is set as 100% viability and the emission rates of treated cells are set in relation to the values of untreated cells. Viabilities are expressed as % values. The corresponding IC₅₀ values of the compounds for cytotoxic activity are determined from the concentration-effect curves by means of non-linear regression.

The anti-proliferative / cytotoxic activity of Examples 1 to 4, 8 to 10, and 11 in A549 NSCLC cell line is determined in the range of IC₅₀= 6.2µM to 0.313µM, and in the HeLa cervical carcinoma cell line in the range of IC₅₀ = 4.2µM to 0.23µM.

### Apoptosis induction

The induction of apoptosis is measured by using the cell death detection ELISA (Art No. 1774425, Roche Biochemicals, Mannheim, Germany). A549 NSCLC cells are seeded into 96 well flat bottom plates at a density of 3x10 E3 cells/well in a total volume of 200µl/well. 24 hours after seeding, 1 µl each of the compound dilutions in DMEM are added in a total volume of 200µl into each well (final volume 200µl/well). Each compound dilution is tested at least as triplicates. Wells containing untreated control cells are filled with 200µl DMEM containing 0.5 vol% DMSO. The cells are incubated with test compound for 48 hours at 37°C in a humidified atmosphere containing 5% carbon dioxide. As a positive control for the induction of apoptosis, cells are treated with 50µM Cisplatin (Gry Pharmaceuticals, Kirchzarten, Germany). Medium is then removed and the cells lysed in 200 µl lysis buffer. After centrifugation as described by the manufacturer, 10 µl of cell lysate is processed as described in the protocol. The degree of apoptosis is calculated as follows: The absorbance at 405 nm obtained with lysates from cells treated with 50µM cisplatin is set as 100 cpu (cisplatin units), while an absorbance at 405 nm of 0.0 is set as 0.0 cpu. The degree of apoptosis is expressed as cpu in relation to the value of 100 cpu reached with the lysates obtained from cells treated with 50µM cisplatin. Apoptosis induction in A549 cells is 310cpu and 46cpu at 10 µM for Example 1 and Example 2, respectively.

## Claims

1. Compounds of formula I or a solvate thereof 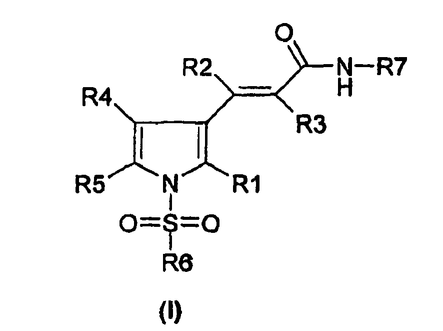 in which
R1 is hydrogen, 1-4C-alkyl, halogen, or 1-4C-alkoxy,
R2 is hydrogen or 1-4C-alkyl,
R3 is hydrogen or 1-4C-alkyl,
R4 is hydrogen, 1-4C-alkyl, halogen, or 1-4C-alkoxy,
R5 is hydrogen, 1-4C,alkyl, halogen, or 1-4C-alkoxy.
R6 is -T1-Q1, in which
T1 Is a bond, or 1-4C-alkylene,
Q1 is naphthyl, HAR, or R61- and/or R62-substituted AR, In which
AR is naphthyl, or HAR, in which
HAR is a monocyclic or fused bicyclic 6- to 10-membered unsaturated heteroaromatic ring comprising one to three heteroatoms, each of which is selected from the group consisting of nitrogen, oxygen and sulfur,
R61 is 1-4C-alkyl, or -T2-N(R811)R612, in which
either
T2 is a bond, and
R611 is hydrogen, 1-4C-alkyl, hydroxy-2-4C-alkyl, 1-4C-alkoxy-2-4C-alkyl, phenyl-1-4C-alkyl, or Har1-1-4C-alkyl, in which
Har1 is optionally substituted by R6111 and/or R6112, and is a monocyclic or fused bicyclic 5- to 10-membered unsaturated heteroaromatic ring comprising one to three heteroatoms, each of which is selected from the group consisting of nitrogen, oxygen and sulfur, in which
R6111 is halogen, or 1-4C-alkyl,
R6112 is 1-4C-alkyl, and
R612 is hydrogen, 1-4C-alkyl, 1-4C-alkoxy-2-4C-alkyl or hydroxy-2-4C-alkyl,
or R611 and R612 together and with inclusion of the nitrogen atom, to which they are bonded, form a heterocyclic ring Het1, in which
Het1 Is morpholino, thiomorpholino, S-oxo-thiomorpholino, S,S-dioxo-thiomorpholino, piperidino, pyrrolidino, piperazino, or 4N-(1-4C-alkyl)-piperazino,
or
T2 is 1-4C-alkylene, or 2-4C-alkylene interrupted by oxygen, and
R611 is hydrogen, 1-4C-alkyl, hydroxy-2-4C-alkyl, 1-4C-alkoxy-2-4C-alkyl, phenyl-1-4C-alkyl, or Har1-1-4C-alkyl, In which
Har1 is optionally substituted by R6111 and/or R6112, and is a monocyclic or fused bicyclic 5- to 10-membered unsaturated heteroaromatic ring comprising one to three heteroatoms, each of which is selected from the group consisting of nitrogen, oxygen and sulfur, in which
R6111 is halogen, or 1-4C-alkyl,
R6112 is 1-4C-alkyl, and
R612 is hydrogen, 1-4C-alkyl, 1-4C-alkoxy-2-4C-alkyl or hydroxy-2-4C-alkyl,
or R611 and R612 together and with inclusion of the nitrogen atom, to which they are bonded, form a heterocyclic ring Het1, in which
Het1 is morpholino, thiomorpholino, S-oxo-thiomorpholino, S,S-dioxo-thiomorpholino, piperidino, pyrrolidino, piperazino, 4N-(1-4Calkyl)-piperazino, imidazolo, pyrrolo, triazolo or pyrazolo,
R62 is 1-4C-alkyl, 1-4C-alkoxy, halogen, cyano, 1-4C-alkoxy-1-4C-alkyl, 1-4C-alkylcarbonylamino or 1-4C-aikylsulphonylamino,
R7 is hydroxyl, or Cyc1, in which
Cyc1 is a ring system of formula la 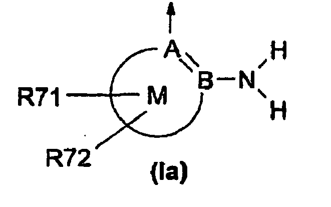
In which
A is C (carbon),
B is C (carbon),
R71 is hydrogen, halogen, 1-4C-alkyl, or 1-4C-alkoxy,
R72 is hydrogen, halogen, 1-4C-alkyl, or 1-4C-alkoxy,
M with inclusion of A and B is either a ring Ar2 or a ring Har2, in which
Ar2 is a benzene ring,
Har2 is a monocyclic 5- or 6-membered unsaturated heteroaromatic ring comprising one to three heteroatoms, each of which is selected from the group consisting of nitrogen, oxygen and sulfur,
and the salts of these compounds, wherein halogen is selected from a group comprising bromine, chlorine and fluorine.

2. Compounds of formula I according to claim 1,
in which
R1 is hydrogen, or 1-4C-alkyl,
R2 is hydrogen, or 1-4C-alkyl,
R3 is hydrogen, or 1-4C-alkyl,
R4 is hydrogen, or 1-4C-alkyl,
R5 is hydrogen, or 1-4C-alkyl,
R6 is -T1-Q1, In which
T1 is a bond,
Q1 is naphthyl, HAR, R61-substituted AR, R62-substituted AR, or R61- and R62-substituted AR, in which
AR is naphthyl, or HAR, in which
HAR is either
a monocyclic 5-membered unsaturated heteroaromatic ring comprising one, two or three heteroatoms, each of which is selected from the group consisting of nitrogen, oxygen and sulfur, or
a monocyclic 6-membered unsaturated heteroaromatic ring comprising one or two nitrogen atoms, or
a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,
R61 is 1-4C-alkyl, or-T2-N(R611)R612, in which
T2 is a bond or 1-4C-alkylene,
R611 is hydrogen, 1-4C-alkyl, phenyl-1-4C-alkyl, or Har1-1-4C-alkyl, in which
Har1 is either
a monocyclic 5-membered unsaturated heteroaromatic ring comprising one, two or three heteroatoms, each of which is selected from the group consisting of nitrogen, oxygen and
sulfur, or a monocyclic 6-membered unsaturated heteroaromatic ring comprising one or two nitrogen
atoms, or a fused bicyclic 9-membered unsaturated heteroaromatic ring comprising one, two or three heteroatoms, each of which is selected from the group consisting of nitrogen, oxygen and sulfur, or
a fused bicyclic 10-membered unsaturated heteroaromatic ring comprising one or two heteroatoms, each of which is selected from the group consisting of nitrogen, oxygen and sulfur,
R612 is hydrogen, 1-4C-alkyl, or hydroxy-2-4C-alkyl,
or R611 and R612 together and with inclusion of the nitrogen atom, to which they are bonded, form a heterocyclic ring Het1, in which
Het1 is morpholino, piperidino, pyrrolidino, piperazino, or 4N-methyl-piparazino,
R62 is 1-4C-alkyl, 1-4C-alkoxy, or halogen,
R7 is hydroxyl, or 2-aminophenyl,
and the salts of these compounds.

3. Compounds of formula I according to claim 1, in which
R1 is hydrogen,
R2 is hydrogen,
R3 is hydrogen,
R4 is hydrogen,
R5 is hydrogen,
R6 is -T1-Q1, in which
T1 is a bond,
Q1 is naphthyl, HAR, R61-substituted AR, N-methyl-imidazolyl, N-methyl-pyrazotyl, N-methyl- indolyl, mono- or di-methyl-substituted thiazolyl, methyl -substituted N-methyl-imidazolyl, or methyl substituted N-methyl-pyrazolyl, in which
AR is naphthyl, or HAR, in which
HAR is pyridinyl, thiazolyl, benzothiophenyl, benzothiazolyl, benzofuranyl, indolyl, quinolinyl or isoquinolinyl,
R61 is 1-4C-alkyl, or -T2-N(R611)R612, in which
T2 is a bond or 1-2C-alkylene,
R611 is hydrogen or 1-4C-alkyl,
R612 is hydrogen or 1-4C-alkyl,
or R611 and R612 together and with inclusion of the nitrogen atom, to which they are bonded, form a heterocyclic ring Het1, in which
Het1 is morpholino, piperidino, pyrrolidino, piperazino, or 4N-methyl-piperazino,
R7 is hydroxyl, or 2-aminophenyl,
and the salts of these compounds.

4. Compounds of formula I according to claim 1, in which
R1 Is hydrogen,
R2 is hydrogen,
R3 is hydrogen,
R4 is hydrogen,
R5 is hydrogen,
R6 is T1-Q1, in which
T1 is a bond,
Q1 is naphthyl, HAR, R61-substituted pyridinyl, N-methyl-imidazolyl, N-methyl-pyrazolyl, mono- or di-methyl-substituted thiazolyl, methyl -substituted N-methyl-imidazolyl, or methyl substituted N-methyl-pyrazolyl, in which
HAR is pyridinyl, thiazolyl, benzothiophenyl or benzothiazolyl,
R61 is -T2-N(R611)R612, in which
T2 is a bond or 1-2C-alkylene,
R611 is hydrogen or 1-2C-alkyl,
R612 is hydrogen or 1-2C-alkyl,
or R611 and R612 together and with inclusion of the nitrogen atom, to which they are bonded, form a heterocyclic ring Het1, in which
Het1 is morpholino, piperidino, pyrrolidino, piperazino, or 4N-methyl-piperazino,
R7 is hydroxyl, or 2-aminophenyl,
and the salts of these compounds.

5. Compounds of formula I according to claim 1, in which
R1 is hydrogen,
R2 is hydrogen,
R3 is hydrogen,
R4 is hydrogen,
R5 is hydrogen,
R6 is-T1-Q1, in which
T1 is a bond,
Q1 is naphthyl, HAR, 2-(R61)-pyridin-3-yl, 1-methyl-pyrazol-4-yl, 1-methyl-imidazol-4-yl, 1,2- dimethyl-imidazol-4-yl, or 2,4-dimethyl-thiazol-5-yl, in which
HAR is pyridin-3-yl, benzothiophen-2-yl or benzothiazol-6-yl,
R61 is -T2-N(R611)R612, in which
T2 is a bond or methylene,
R611 is hydrogen or methyl,
R612 is hydrogen or methyl,
or R611 and R612 together and with inclusion of the nitrogen atom, to which they are bonded, form a heterocyclic ring Het1, in which
Het1 is morpholino,
R7 is hydroxyl, or 2-aminophenyl,
and the salts of these compounds.

6. Compounds of formula I according to claim 1, in which
R1 is hydrogen,
R2 is hydrogen,
R3 is hydrogen,
R4 is hydrogen,
R5 is hydrogen,
R6 is-T1-Q1, in which
T1 is a bond,
Q1 is naphthyl, HAR, or 2-(R61)-pyridin-3-yl, in which
HAR is pyridinyl,
R61 is -T2-N(R611)R612, in which
T2 is a bond or methylene,
R611 is hydrogen or methyl,
R612 is hydrogen or methyl,
or R611 and R612 together and with inclusion of the nitrogen atom, to which they are bonded, form a heterocyclic ring Het1, in which
Het1 is morpholino,
R7 is hydroxyl, or 2-aminophenyl,
and the salts of these compounds.

7. A compound of formula I according to claim 1 which is selected from
(E)-N-Hydroxy-3-[1-(naphthalene-2-sulfonyl)-1H-pyrrol-3-yl]-acrylamide,
(E)-N-(2-Amlno-phenyl)-3-[1-(naphthalene-2-sulfonyl)-1H-pyrrol-3-yl]-acrylamide,
(E)-N-Hydroxy-3-[1-(pyridine-3-sulfonyl)-1H-pyrrol-3-yl]acrylamide,
(E)-N-Hydroxy-3-[1-(6-morpholin-4-yl-pyridine-3-sulfonyl)-1H-pyrrol-3-yl]-acrylamide,
(E)-N-(2-Amino-phenyl)-3-[1-(benzo[b]thiophene-2-sulfonyl)-1H-pyrrol-3-yl]-acrylamide,
(E)-N-(2-Amino-phenyl)-3-[1-(benzothiazole-6-sulfonyl)-1H-pyrrol-3-yl]-acrylamide,
(E)-N-Hydroxy-3-[1-(1-methyl-1H-imidazole-4-sulfonyl)-1H-pyrrol-3-yl]-acrylamide,
(E)-N-Hydroxy-3-[1-(1-methyl-1H-pyrazole-4-sulfonyl)-1H-pyrrol-3-yl]-acrylamide,
(E)-3-[1-(Benzo[b]thiophene-2-sulfonyl)-1H-pyrrol-3-yl]-N-hydroxy-acrylamide,
(E)-3-[1-(Benzothiazole-6-sulfonyl)-1H-pyrrol-3-yl]-N-hydroxy-acrylamide,
(E)-3-[1-(2,4-Dimethyl-thiazole-5-sulfonyl)-1H-pyrrol-3-yl]-N-hydroxy-acrylamide, and
(E)-3-[1-(1,2-Dimethyhl-1H-imidazole-4-sulfonyl)-1H-pyrrol-3-yl]-N-hydroxy-acrylamide,
or a salt thereof.

8. Compounds as claimed in any of the claims 1 to 7 for use in the treatment of diseases.

9. A pharmaceutical composition comprising one or more compounds as claimed any of the claim 1 to 7 together with customary pharmaceutical excipients and/or auxiliaries.

10. Use of compounds as claimed in any of the claims 1 to 7 for the manufacture of pharmaceutical compositions for treating diseases responsive or sensitive to inhibition of histone deacetylase activity.

11. Use of compounds as claimed any of the claims 1 to 7 for the manufacture of pharmaceutical compositions for treatring benign and/or malignant neoplasia, such as e.g. cancer.

12. A compound as claimed in any of the claims 1 to 7 for treating, preventing or ameliorating hyperproliferative diseases of benign or malignant behaviour and/or disorders responsive to induction of apoptosis, such as, for example, benign or malignant neoplasia, e.g. cancer, in a patient.

13. A compound as claimed in any of the claims 1 to 7 for treating benign and/or malignant neoplasia, such as e.g. cancer, in a patient comprising administering to said patient a therapeutically effective and tolerable amount of said compound optionally, simultaneously, sequentially or separately with one or more further therapeutic agents.

14. A combination comprising
a first active ingredient, which is at least one compound according to any of the claims 1 to 7, and a second active ingredient, which is at least one anti-cancer agent selected from the group consisting of chemotherapeutic anti-cancer agents and target-speciffic anti-cancer agents,
for separate, sequential, simultaneous, concurrent or chronologically staggered use in therapy, such as e.g. In therapy of benign or malignant neoplasia, e.g. cancer.

15. A compound as claimed in any of the claims 1 to 7 for treating, preventing or ameliorating hyperproliferative diseases and/or disorders responsive to induction of apoptosis, such as, for example, benign or malignant neoplasia, e.g. cancer, in a patient comprising administering separately, simultaneously, concurrently, sequentially or chronologically staggered to said patient in need thereof
an amount of a first active compound, which is said compound according to any of the claims 1 to 7, and an amount of at least one second active compound, said second active compound being an anti-cancer agent selected from the group consisting of chemotherapeutic anti-cancer agents and target-specific anti-cancer agents,
wherein the amounts of the first active compound and said second active compound result in a therapeutic effect.

16. The combination or a compound according to claim 14 or 15, for treating, preventing or ameliorating hyperproliferative diseases and or disorders responsive to induction of apoptosis in which said chemotherapeutic anti-cancer agents are selected from (i) alkylating/carbamylating agents including Cyclophosphamid. Ifosfamid, Thiotepa, Melphalan and chloroethylnitrosourea: (ii) platinum derivatives including cis-platin, oxaliplatin and carboplatin; (III) antimitotic agents /tubulin inhibitors including vinca alkaloids, such as e.g. vincristine, vinblastine or vinorelbine, taxanes, such as e.g. Paclitaxel, Docetaxel and analogs as well as formulations and conjugates thereof, and epothilones, such as e.g. Epothilons B, Azaepothilone or ZK-EPO; (IV) topolsomerase inhibitors including anthracyclines, such as e.g. Doxorubicin, epipodophyllotoxines, such as e.g. Etoposide, and camptothecin and camptothecin analogs, such as e.g. ininotecan or Topotecan, (V) pyrimidine antagonists including 5-fluorouracil. Capecitabine, Arabinosylcytosine/Cytarabin and Gemcitabine; (VI) purin antagonists including 6-mercaptopurine, 6-thioguanine and fludarabine; and (vii) folle acid antagonists including methotrexate and pemetrexed.

17. The combination or a compound according to claim 14, 15 or 16 for treating, preventing or ameliorating hyperproliferative diseases and or disorders responsive to induction of apoptosis, in which said target-specific anti-cancer agents are selected from (i) kinase inhibitors including imatinib, ZO.1839 / Gefitinib, BAY43-9006 / Sorafenib, SU11248/Sunitinib and OSI-774 / Erlotinib; (il) proteasome inhibitors including PS-341/Bortezomitor; (iii) histone deacetylase inhibitors including SAHA, PXD101, MS275, MQCD0103, Depsipeptide / FK228, NVP-LBH589, NVP-LAQB24, Valproic acid (VPA) and butyrates; (iv) heat shock protein 90 inhibitors including 17-allylaminogeldanamycin (17-AAG); (v) vascular targeting agents (VAT) including combretastatin A4 phosphate and AVE8062/AC7700, and anti-angiogenic drugs including VEGF antibodies, such as e.g. Bevacizumab, and KDR tyrosine kinase inhibitors, such as e.g. PTK787 / ZK222684 (Vatalanib); (vi) monoclonal antibodies including Trastuzumab, Rituximab, Alemtuzumab, Tositumab, Cetuximab and Bevacizumab as well as mutants and conjugates or monoclonal antibodies, such as e.g. Gemtuzumab ozogamicin or ibritumomab tiuxetan, and antibody fragments: (vii) oligonucleotide based therapeutics including G-3138 / Oblimersen; (viii) TolHike receptor/ TLR 9 agonists including Promune®, TLR 7 agonists including imiquimod and isatoribine and analogues thereof, or TLR 7/8 agonist including Resiquimod as well as immunostimulatory RNA as TLR 7/8 agonists; (ix) protease inhibitors (x) hormonal therapeutics including anti-estrogens, such as e.g. Tamoxifen or Raloxifen, anti-androgens, such as e.g. Flutamide or Casodex, LHRH analogs, such as e.g. Luprolide, Goserelin or Triptorelin, and aromatase inhibitors;
bleomycin; retinolds including all-trans retinoic acid (ATRA); DNA methyltransferase inhibitors including the 2-deoxycytidine derivative Decitabine and 5-Azacytidine; atanosine; cytokines including interleukin-2; interferons including interferon α2 and interferon-γ: and death receptor agonists including TRAIL, DR4/5 agonistic antibodies, FasL and TNF-R agonists.

18. The use, a compound or combination according to any of the claims 11 to 15 for treating, preventing or ameliorating hyperproliferative diseases and or disorders responsive to induction of apoptosis, in which said cancer is selected from the group consisting of
cancer of the breast, bladder, bone, brain, central and peripheral nervous system, colon, endocrine glands, esophagus, endometrium, germ cells, head and neck, iddney, liver, lung, larynx and hypopharynx, mesothelloma, sarcoma, ovary, pancreas, prostate, rectum, renal, small intestine, soft tissue, testis, stomach, skin, ureter, vagina and vulva;
inherited cancers, retinomblastoma and Wilma tumor,
leukemia, lymphome, non-Hodgkins disease, chronic and acute myeloid leukaemia, acute lymphoblastic leukemia, Hodgkins disease, multiple myeloma and T-cell lymphoma;
myelodysplastic syndrome, plasma cell neoplasia, paraneoplastic syndromes, cancers of unknown primary site and AIDS related malignancies.

19. Use of compounds as claimed in any of the claims 1 to 7 for the manufacture of pharmaceutical compositions for treating diseases different to malignant neoplasia, such as e.g. arthropathies and osteopathological diseases, autoimmune diseases including transplant rejection, acute and chronic inflammatory diseases, hyperproliferative diseases or neuropathological disorders.

## Patentansprüche

1. Verbindungen der Formel I oder eines Solvates davon 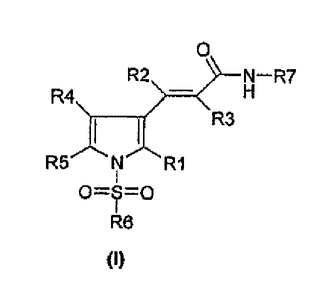 wobei
R1 für Wasserstoff, 1-4C-Alkyl, Halogen oder 1-4C-Alkoxy steht,
R2 für Wasserstoff oder 1-4C-Alkyl steht,
R3 für Wasserstoff oder 1-4C-Alkyl steht,
R4 für Wasserstoff, 1-4C-Alkyl, Halogen oder 1-4C-Alkoxy steht,
R5 für Wasserstoff, 1-4C-Alkyl, Halogen oder 1-4C-Alkoxy steht,
R6 für -T1-Q1 steht, wobei
T1 eine Bindung oder 1-4C-Alkylen darstellt,
Q1 für Naphthyl, HAR oder mit R61- und/ oder R62substituiertes AR steht, wobei
AR Naphthyl oder HAR darstellt, wobei
HAR für einen monozyklischen oder kondensierten bizyklischen 5- bis 10-gliedrigen, ungesättigten, heteroaromatischen Ring steht, der ein bis drei Heteroatome umfasst, von denen ein jedes aus der aus Stickstoff, Sauerstoff und Schwefel bestehenden Gruppe ausgewählt wurde.
R61 für ein 1-4C-Alkyl oder -T2-N(R611) R612 steht, wobei
entweder
T2 eine Bindung darstellt und
R511 für Wasserstoff, 1-4C-Alkyl, Hydroxy-2-4C-Alkyl, 1-4C-Alkoxy-2-4C-Alkyl, Phenyl-1-4C-Alkyl oder Harl-1-4C-Alkyl steht, wobei
Har1 optional durch R6111 und/ oder R6112 substituiert ist und für einen monozyklischen oder kondensierten bizyklischen 5- bis 10-gliedrigen, ungesättigten, heteroaromatischen Ring steht, der ein bis drei Heteroatome umfasst, von denen ein jedes aus der aus Stickstoff, Sauerstoff und Schwefel bestehenden Gruppe ausgewählt wurde, wobei
R6111 für ein Halogen oder 1-4C-Alkyl steht,
R6112 für ein 1-4C-Alkyl steht und
R612 für Wasserstoff, 1-4C-Alkyl, 1-4C-Alkoxy-2-4C-Alkyl oder Hydroxy-2-4C-Alkyl steht,
oder R611 und R612 gemeinsam und zusammen mit dem Stickstoffatom, an das sie gebunden sind, einen heterozyklischen Ring Het1 bilden, wobei
Het 1 für Morpholin, Thiomorpholin, S-Oxo-Thiomorpholin, S,S-Dioxothiomorpholin, Piperidin, Pyrrolidin, Piperazin oder 4N-1(1-4C-Alkyl)-Piperazin steht
oder
T2 für 1-4C-Alkylen oder durch Sauerstoff unterbrochenes 2-4C-Alkylen steht und
R611 für Wasserstoff, 1-4C-Alkyl, Hydroxy-2-4C-Alkyl, 1-4C-Alkoxy-2-4C-Alkyl, Phenyl-1-4C-Alkyl oder Har1-1-4C-Alkyl steht, wobei
Har1 optional durch R6111 und/ oder R6112 substituiert ist und einen monozyklischen oder kondensierten bizyklischen 5- bis 10-gliedrigen, ungesättigten, heteroaromatischen Ring darstellt, der ein bis drei Heteroatome umfasst, von denen ein jedes aus der aus Stickstoff, Sauerstoff und Schwefel bestehenden Gruppe ausgewählt wurde, wobei
R6111 für Halogen oder 1-4C-Alkyl steht,
R6112 für 1-4C-Alkyl steht und
R612 für Wasserstoff, 1-4C-Alkyl, 1-4C-Alkoxy-2-4C-Alkyl oder Hydroxy-2-4C-Alkyl steht,
oder R611 und R612 gemeinsam und zusammen mit dem Stickstoffatom, an das sie gebunden sind, einen heterozyklischen Ring Het1 bilden, wobei
Het 1 für Morpholin, Thiomorpholin, S-Oxo-Thiomorpholin, S,S-Dioxothiomorpholin, Piperidin, Pyrrolidin, Piperazin, 4N- (1-4C-Alkyl)-Piperazin, Imidazol, Pyrrol, Triazol oder Pyrazol steht,
R62 für 1-4C-Alkyl, 1-4C-Alkoxy, Halogen, Cyan, 1-4C-Alkoxy-1-4C-Alkyl, 1-4C- Alkylcarbonylamin oder 1-4C-Alkylsulphonylamin steht
R7 für Hydroxyl oder Cyc1 steht, wobei
Cyc1 ein Ringformelsystem Ia ist 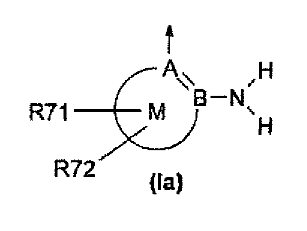
wobei
A für C (Kohlenstoff) steht
B für C (Kohlenstoff) steht,
R71 für Wasserstoff, Halogen, 1-4C-Alkyl oder 1-4C-Alkoxy steht,
R72 für Wasserstoff, Halogen, 1-4C-Alkyl oder 1-4C-Alkoxy steht,
M zusammen mit A und B entweder einen Ring Ar2 oder einen Ring Har2 darstellt, wobei
Ar2 einen Benzenring darstellt,
Har2 für einen monozyklischen, 5- oder 6-gliedrigen, ungesättigten, heteroaromatischen Ring steht, der ein bis drei Heteroatome umfasst, von denen ein jedes aus der aus Stickstoff, Sauerstoff und Schwefel bestehenden Gruppe ausgewählt wurde, und die Salze dieser Verbindungen, wobei das Halogen aus einer aus Brom, Chlor und Fluor bestehenden Gruppe ausgewählt wird.

2. Verbindungen der Formel I nach Anspruch 1,
wobei
R1 für Wasserstoff oder 1-4C-Alkyl steht,
R2 für Wasserstoff oder 1-4C-Alkyl steht,
R3 für Wasserstoff oder 1-4C-Alkyl steht,
R4 für Wasserstoff oder 1-4C-Alkyl steht,
R5 für Wasserstoff oder 1-4C-Alkyl steht,
R6 für T1-Q1 steht, bei dem
T1 eine Bindung darstellt,
Q1 für Naphthyl, HAR mit R61 substituiertes AR, mit R62 substituiertes AR, oder mit R61 und R62 substituiertes AR steht, wobei
AR für Naphthyl oder HAR steht, wobei
HAR entweder
für einen monozyklischen 5-gliedrigen, ungesättigten, heteroaromatischen Ring steht, der ein, zwei oder drei Heteroatome umfasst, von denen ein jedes aus der aus Stickstoff, Sauerstoff und Schwefel bestehen Gruppe ausgewählt wurde oder
für einen monozyklischen 6-gliedrigen, ungesättigten, heteroaromatischen Ring steht, der ein oder zwei Stickstoffatome umfasst oder
für einen kondensierten bizyklischen 9-gliedrigen, ungesättigten, heteroaromatischen Ring steht, der ein, zwei oder drei Heteroatome umfasst, von denen ein jedes aus der aus Stickstoff, Sauerstoff und Schwefel bestehenden Gruppe ausgewählt wurde oder für einen kondensierten bizyklischen 10-gliedrigen, ungesättigten,
heteroaromatischen Ring steht, der ein oder zwei Heteroatome umfasst, von denen ein jedes aus der aus Stickstoff, Sauerstoff und Schwefel bestehenden Gruppe ausgewählt wurde,
R61 für ein 1-4C-Alkyl oder -T2-N(R611) R612 steht, wobei
T2 eine Bindung oder 1-4C-Alkylen darstellt,
R611 für Wasserstoff, 1-4C-Alkyl, Phenyl-1-4C-Alkyl oder Har1-1-4C-Alkyl steht, wobei
Har1 entweder
für einen monozyklischen 5-gliedrigen, ungesättigten, heteroaromatischen Ring steht, der ein, zwei oder drei Heteroatome umfasst, von denen ein jedes aus der aus Stickstoff, Sauerstoff und Schwefel bestehen Gruppe ausgewählt wurde oder für einen monozyklischen 6-gliedrigen, ungesättigten, heteroaromatischen Ring steht, der ein oder zwei Stickstoffatome umfasst oder
für einen kondensierten bizyklischen 9-gliedrigen, ungesättigten, heteroaromatischen Ring steht, der ein, zwei oder drei Heteroatome umfasst, von denen ein jedes aus der aus Stickstoff, Sauerstoff und Schwefel bestehenden Gruppe ausgewählt wurde oder für einen kondensierten bizyklischen 10-gliedrigen, ungesättigten,
heteroaromatischen Ring steht, der ein oder zwei Heteroatome umfasst, von denen ein jedes aus der aus Stickstoff, Sauerstoff und Schwefel bestehenden Gruppe ausgewählt wurde,
R612 für Wasserstoff, 1-4C-Alkyl oder Hydroxy-2-4C-Alkyl steht,
oder R611 und R612 gemeinsam und zusammen mit dem Stickstoffatom, an das sie gebunden sind, einen heterozyklischen Ring Het1 bilden, wobei
Het 1 für Morpholin, Piperidin, Pyrrolidin, Piperazin oder 4N-Methyl-Piperazin steht,
R62 für ein 1-4C-Alkyl, 1-4C-Alkoxy oder Halogen steht,
R7 für Hydroxyl oder 2-Aminophenyl steht,
und die Salze dieser Verbindungen.

3. Verbindungen der Formel I nach Anspruch 1, wobei
R1 für Wasserstoff steht,
R2 für Wasserstoff steht,
R3 für Wasserstoff steht,
R4 für Wasserstoff steht,
R5 für Wasserstoff steht,
R6 für T1-Q1 steht, bei dem
T1 eine Bindung darstellt,
Q1 für Naphthyl, HAR, mit R61 substituiertes AR, N-Methyl-Imidazolyl, N-Methyl- Pyrazolyl, N-Methyl-Indolyl, mit Mono- oder Dimethylsubstituiertes Thiazolyl, methylsubstituiertes N-Methyl-Imidazolyl oder methylsubstituiertes N-Methyl- Pyrazolyl steht, wobei
AR für Naphthyl oder HAR steht, wobei
HAR für Pyridinyl, Thiazolyl, Benzothiophenyl, Benzothiazolyl, Benzofuranyl, Indolyl, Chinolinyl oder Isochinolinyl steht
R61 für 1-4C-Alkyl oder -T2-N(R611)R612 steht, wobei
T2 eine Bindung oder 1-2C-Alkylen darstellt,
R611 für Wasserstoff oder 1-4C-Alkyl steht,
R612 für Wasserstoff oder 1-4C-Alkyl steht
oder R611 und R612 gemeinsam und zusammen mit dem Stickstoffatom, an das sie gebunden sind, einen heterozyklischen Ring Het1 bilden, wobei
Het 1 für Morpholin, Piperidin, Pyrrolidin, Piperazin oder 4N-Methyl-Piperazin steht,
R7 für Hydroxyl oder 2-Aminophenyl steht,
und die Salze dieser Verbindungen

4. Verbindungen der Formel I nach Anspruch 1,
wobei
R 1 für Wasserstoff steht,
R2 für Wasserstoff steht,
R3 für Wasserstoff steht,
R4 für Wasserstoff steht,
R5 für Wasserstoff steht,
R6 für T1-Q1 steht, wobei
T1 eine Bindung darstellt,
Q1 für Naphthyl, HAR, mit R61 substituiertes Pyridinyl, N-Methyl-Imidazolyl, N- Methyl-Pyrazolyl, Mono- oder Dimethyl substituiertes Thiazolyl, mit Methyl substituiertes N-Methyl-Imidazolyl oder mit Methyl substituiertes N-Methyl- Pyrazolyl, wobei
HAR für Pyridinyl, Thiazolyl, Benzothiophenyl oder Benzothiazolyl steht
R61 für-T2-N(R611)R612 steht, wobei
T2 eine Bindung oder 1-2C-Alkylen darstellt,
R611 für Wasserstoff oder 1-2C-Alkyl steht,
R612 für Wasserstoff oder 1-2C-Alkyl steht
oder R611 und R612 gemeinsam und zusammen mit dem Stickstoffatom, an das sie gebunden sind, einen heterozyklischen Ring Het1 bilden, wobei
Het 1 für Morpholin, Piperidin, Pyrrolidin, Piperazin oder 4N-Methyl-Piperazin steht,
R7 für Hydroxyl oder 2-Aminophenyl steht,
und die Salze dieser Verbindungen.

5. Verbindungen der Formel I nach Anspruch 1,
wobei
R1 für Wasserstoff steht,
R2 für Wasserstoff steht,
R3 für Wasserstoff steht,
R4 für Wasserstoff steht,
R5 für Wasserstoff steht,
R6 für T1-Q1 steht, wobei
T1 eine Bindung darstellt,
Q1 für Naphthyl, HAR, 2-(R61)-Pyridin-3-yl, 1-Methyl-Pyrazol-4-yl, 1-Methyl- Imidazol-4-yl, 1,2-Dimethyl-Imidazol-4-yl oder 2,4-Dimethyl-Thiazol-5-yl steht, wobei
HAR für Pyridin-3-yl, Benzothiophexz-2-yl oder Benzothiazol-6-yl steht
R61 für -T2-N(R61 1)R612 steht, wobei
T2 eine Bindung oder Methylendarstellt,
R611 für Wasserstoff oder Methyl steht,
R612 für Wasserstoff oder Methyl steht
oder R611 und R612 gemeinsam und zusammen mit dem Stickstoffatom, an das sie gebunden sind, einen heterozyklischen Ring Het1 bilden, wobei
Het 1 für Morpholin steht,
R7 für Hydroxyl oder 2-aminophenyl steht,
und die Salze dieser Verbindungen.

6. Verbindungen der Formel I nach Anspruch 1,
R1 für Wasserstoff steht,
R2 für Wasserstoff steht,
R3 für Wasserstoff steht,
R4 für Wasserstoff steht,
R5 für Wasserstoff steht,
R6 für T1-Q1 steht, bei dem
T1 eine Bindung darstellt,
Q1 für Naphthyl, HAR oder 2-(R61)-Pyridin-3-yl steht, wobei
HAR für Pyridinyl steht
R61 für -T2-N(R611)R612 steht, wobei
T2 eine Bindung oder Methylen darstellt,
R611 für Wasserstoff oder Methyl steht,
R612 für Wasserstoff oder Methyl steht
oder R611 und R612 gemeinsam und zusammen mit dem Stickstoffatom, an das sie gebunden sind, einen heterozyklischen Ring Het1 bilden, bei dem
Het 1 für Morpholin steht,
R7 für Hydroxyl oder 2-Aminophenyl steht,
und die Salze dieser Verbindungen.

7. Eine Verbindung der Formel I nach Anspruch 1, welche ausgewählt wird aus
(E)-N-Hydroxy-3 - [1-(Naphtalin-2-sulfonyl)-H-pyrrol- -yl] -acrylamide,
(E)-N-(2-Aminopherzyl)-3-[1-(Naphtalin-2-sulfonyl)-1H-pyrrol-3-yl]-acrylamid,
(E)-N-Hydroxy-3-[[Pyridin-3-sulfonyl)-1H-pyrrol-3-yl]-acrylamid,
(E)-N-Hydroxy-3-[1-(6-Morpholin-4-yl-pyridin-3-sulfonyl)-1H-pyrrol-3-yl]-acrylamid,
(E)-N-(2-Aminophenyl)-3-[1-(benzo[b]thiophen-2-sulfonyl)-1H-pyrrol-3-yl]-acrylamid
(E)-N-(2-Aminophenyl)-3-[l-(benzothiazol-6-sulfonyl)- H-pyrrol-3-yl]-acrylamid,
(E)-N-Hydroxy-3-1-(1-Methyl-1H-imidazol-4-sulifonyl)-1H-pyrrol-3-yl]-acrylamid,
(E)-N-Hydroxy-3-1(1 -Methyl- 1H-pyrazol-4-sulfonyl)-1H-pyrrol-3-yl]-acrylamid,
(E)-3 -[1-(Benzo[b]thiophen-2-sulfonyl)-1H-pyrrol-3-yl]-N-hydroxy-acrylamid,
(E)-3-[1-(Benzothiazol-6-sulfonyl)-1H-pyrrol-3-yl]-N-hydroxy-acrylamid,
(E)-3-[1-(2,4-Dimethyl-thiazol-5-sulfonyl)-1H-pyrrol-3-yl]-N-hydroxy-acrylamid und
(E)-3-[1-(1,2-Dimethyl-1H-imidazol-4-sulfonyl)-1H-pyrrol-3 -yl]-N-hydroxy-acrylamid
oder die Salze davon.

8. Verbindungen nach einem der Ansprüche 1 bis 7 zur Verwendung bei der Behandlung von Krankheiten.

9. Eine pharmazeutische Zubereitung, die eine oder mehrere der Verbindungen nach einem der Ansprüche 1 bis 7 zusammen mit handelsüblichen pharmazeutischen Hilfsstoffen und/ oder Zusätzen umfasst.

10. Verwendung von Verbindungen nach einem der Ansprüche 1 bis 7 für die Herstellung von pharmazeutischen Zubereitungen zur Behandlung von Erkrankungen, die auf die Hemmung der Histon-Deacetylase-Aktivität reagieren oder empfindlich reagieren.

11. Verwendung von Verbindungen nach einem der Ansprüche 1 bis 7 zur Herstellung von pharmazeutischen Zubereitungen zur Behandlung benigner und/ oder maligner Neoplasie, wie zum Beispiel Krebs.

12. Eine Verbindung nach einem der Ansprüche 1 bis 7 zur Behandlung, Vorbeugung und Verbesserung von hyperproliferativen Erkrankungen mit benignem oder malignem Verhalten und/ oder Störungen bei einem Patienten, die auf die Induktion von Apoptose reagieren, wie zum Beispiel benigne und maligne Neoplasie, z.B. Krebs.

13. Eine Verbindung nach einem der Ansprüche 1 bis 7 zur Behandlung benigner und/ oder maligner Neoplasie wie z.B. Krebs bei einem Patienten, die die Verabreichung einer therapeutisch wirksamen und verträglichen Menge der genannten Verbindung gegebenenfalls gleichzeitig, nacheinander oder getrennt voneinander mit einem oder mehreren weiteren therapeutischen Wirkstoffen an besagten Patienten umfasst.

14. Eine Kombination, die einen ersten Wirkstoff, der mindestens eine Verbindung nach einem der Ansprüche 1 bis 7 ist, und einen zweiten Wirkstoff, bei dem es sich mindestens um einen Antikrebs-Wirkstoff handelt, welcher aus der Gruppe ausgewählt wurde, die aus chemotherapeutischen Antikrebs-Wirkstoffen und zielgerichteten Antikrebs-Wirkstoffen besteht, umfasst,
für die getrennte, nacheinander erfolgende, gleichzeitige, parallele oder zeitlich gestaffelte Verwendung bei der Therapie, wie z.B. bei der Therapie von benigner oder maligner Neoplasie, z.B. Krebs.

15. Eine Verbindung nach einem der Ansprüche 1 bis 7 für die Behandlung, Verhütung oder Verbesserung von hyperproliferativen Erkrankungen und/ oder Störungen, die auf die Induktion von Apoptose reagieren, wie zum Beispiel benigne und maligne Neoplasie, z.B. Krebs, die die getrennte, gleichzeitige, parallele, nacheinander erfolgende oder zeitlich gestaffelte Verabreichung einer Menge eines ersten Wirkstoffs, bei dem es sich um eine der genannten Verbindungen nach einem der Ansprüche 1 bis 7 handelt, und einer Menge von mindestens einer zweiten wirksamen Verbindung, wobei die genannte zweite wirksame Verbindung ein Antikrebs-Wirkstoff ist, der aus der Gruppe ausgewählt wurde, die aus chemotherapeutischen Antikrebs-Wirkstoffen und zielgerichteten Antikrebs-Wirkstoffen besteht, an besagten Patienten bei Bedarf umfasst, wobei die Mengen des ersten Wirkstoffs und genannter zweiter wirksamer Verbindung zu einer therapeutischen Wirkung führen.

16. Die Kombination oder eine Verbindung nach Anspruch 14 oder 15 zur Behandlung, Verhütung oder Verbesserung hyperproliferativer Erkrankungen und/ oder Störungen, die auf die Induktionvon Apoptose reagieren, bei der besagte chemotherapeutische Antikrebs-Wirkstoffe ausgewählt werden aus (i) Alkylierungs-/Carbamylierungsmitteln, darunter Cyclophosphamid, Ifosfamid, Thiotepa, Melphalan und Chlorethylnitrosourea; (ii) Platinderivaten, darunter cis-Platin, Oxaliplatin und Carboplatin; (iii) antimitotischen Wirkstoffen/ Tubulin-Inhibitoren, darunter Vinca-Alkaloide wie zum Beispiel Vincristin, Vinblastin oder Vinorelbin, Taxane wie zum Beispiel Paclitaxel, Docetaxel und Analoga sowie Formulierungen und Konjugate davon und Epothilone wie zum Beispiel Epothilon B, Azaepothilon oder ZK-EPO; (iv) Topoisomerase-Inhibitoren darunter Anthracycline wie zum Beispiel Doxorubicin, Epipodophyllotoxine wie zum Beispiel Etoposid und Camptothecin und Camptothecin-Analoga wie zum Beispiel Irinotecan oder Topotecan; (v) Pyrimidin-Antagonisten darunter 5-Fluorouracil, Capecitabin, Arabinosylcytosin/Cytarabin und Gemcitabin; (vi) Purin-Antagonisten darunter 6-Mercaptopurir, 6-Thioguanin und Fludarabin und (vii) Folsäure-Antagonisten einschließlich Methotrexat und Pemetrexed.

17. Die Kombination oder eine Verbindung nach Anspruch 14, 15 oder 16 zur Behandlung, Verhütung oder Verbesserung hyperproliferativer Erkrankungen und/ oder Störungen, die auf die Induktionvon Apoptose reagieren, wobei die zielgerichteten Antikrebs-Mittel ausgewählt werden aus (i) Kinase-Inhibitoren darunter Imatinib, ZD-1839 / Gefinitib, BAY43-9006 / Sorafenib, SU1124 / Sunitinib und OSI-774 / Erlotinib; (ii) Proteasom-Inhibitoren darunter PS-341 / Bortezomib; (iii) Histon-Deacetylase-Inhibitoren darunter SAHA, PXD101, MS275, MGCD0103, Depsipeptid / FK228, NVP-LBH589, NVP-LAQ824, Valproinsäure (VPA) und Butyraten; (iv) Hitzeschockprotein-90-Inhibitoren darunter 17-Allylaminogeldanamycin (17-AAG); (v) vaskulären Targetingmitteln (VAT) darunterCombretastatin A4 Phosphat und AVE8062 / AC7700 und Anti-Angiogenese-Mitteln darunterVEGF-Antikörper, wie zum Beispiel Bevacizumab, und KDR-Tyrosinkinase-Inhibitoren, wie zum Beispiel PTK787 /ZK222584 (Vatalanib); (vi) monoklonalen Antikörpern darunter Trastuzumab, Rituximab, Alemtuzumab, Tositumab, Cetuximab und Bevacizumab sowie Mutanten und Konjugaten von monoklonalen Antikörpern, wie zum Beispiel Gemtuzumab Ozogamicin oder Ibritumomab Tiuxetan und Antikörperfragmenten; (vii) Therapeutika auf der Basis von Oligonukleotiden darunter G-3139 / Oblimersen; (viii) Toll-artigen Rezeptoren / TLR 9-Agonisten darunter Pramune®, TLR 7-Agonisten darunter Imiquimod und Isatoribin und Analoga davon oder TLR 7/8-Agonisten darunter Resiquimod sowie immunstimulierenden RNA als TLR 7/8-Agonisten; (ix) Proteaseinhibitoren; (x) hormonellen Therapeutika einschließlich Anti-Östrogenen, wie zum Beispiel Tamoxifen oder Raloxifen, Anti-Androgenen, wie zum Beispiel Flutamid oder Casodex, LHRH-Analoga, wie zum Beispiel Luprolid, Goserelin oder Triptorelin, und Aromatase-Inhibitoren; Bleomycin; Retinoiden einschließlich All-Trans-Retinolsäure (ATRA); DNA-Methyltransferase-Inhibitoren darunter das 2-Deoxycytidin-Derivat Decitabin und 5-Azacytidin; Alanosin; Cytokinen darunter Interleukin-2; Interferonen darunter Interferon α2 und Interferon-γ, und Todesrezeptoragonisten einschließlich TRAIL, DR4/5 agonistischen Antikörpern, FasL und TNF-R-Agonisten.

18. Die Verwendung einer Verbindung oder Kombination nach einem der Ansprüche 11 bis 15, zur Behandlung, Verhütung oder Verbesserung hyperproliferativer Erkrankungen und/ oder Störungen, die auf die Induktionvon Apoptose reagieren, wobei der genannte Krebs ausgewählt ist aus der Gruppe, die besteht aus Brustkrebs, Blasenkrebs, Knochenkrebs, Gehirntumor, Krebs des zentralen oder peripheren Nervensystems, Darmkrebs, Krebs der endokrinen Drüsen, Speiseröhrenkrebs, Endometriumkrebs, Keimzellenkrebs, Krebs an Kopf und Hals, Nierenkrebs, Leberkrebs, Lungenkrebs, Kehlkopfkrebs und Hypopharynxkrebs, Mesotheliom, Sarkom, Eierstockkrebs, Bauchspeicheldrüsenkrebs, Prostatakrebs, Mastdarmkrebs, Nierenkrebs, Dünndarmkrebs, Weichteilkrebs, Hodenkrebs, Magenkrebs, Hautkrebs, Hamleiterkrebs, Krebs der Vagina und Vulva; ererbte Krebsarten, Retinoblastom und Wilms-Tumor;
Leukämie, Lymphom, Non-Hodgkin-Erkrankung, chronischer und akuter myeloischer Leukämie, akuter lymphoblastischer Leukämie, Hodgkin-Erkrankung, multiplem Myelom und T-Zellen-Lymphom;
myelodysplastischem Syndrom, Plasmazellen-Neoplasie, paraneoplastischem Syndrom, Krebs mit unbekanntem Anfangsgebiet und bösartigen Tumoren in Verbindung mit AIDS.

19. Verwendung von Verbindungen gemäß den Anforderungen einer der Ansprüche 1 bis 7 zur Herstellung von pharmazeutischen Zubereitungen zur Behandlung von anderen Erkrankungen als maligner Neoplasie, wie z.B. Gelenkerkrankungen und osteopathologischen Erkrankungen, Autoimmunkrankheiten einschließlich Transplantatabstoßung, akuten und chronischen Entzündungen, hyperproliferativen Erkrankungen oder neuropathologischen Störungen.

## Revendications

1. Composés de la formule 1 ou un solvate de ceux-ci 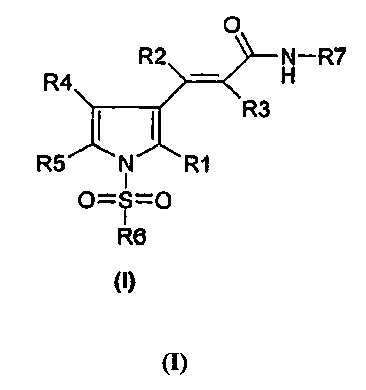 où
R1 représente un hydrogène, alkyle en C1-4, halogène ou alcoxy en C1-4,
R2 représente un hydrogène ou alkyle en C1-4,
R3 représente un hydrogène ou alkyle en C1-4,
R4 représente un hydrogène, alkyle en C1-4, halogène ou alcoxy en C1-4,
R5 représente un hydrogène, alkyle en C1-4, halogène ou alcoxy en C1-4,
R6 représente -T1-Q1, où
T1 représente une liaison ou un alkylène en C1-4,
Q1 représente un naphtyle, HAR ou AR substitué par R61 et/ou R62, où
AR représente un naphtyle ou HAR, où
HAR représente un cycle hétéroaromatique insaturé monocyclique ou bicyclique fusionné comportant 5 à 10 chaînons, comprenant un à trois hétéroatomes, chacun d'eux étant choisi parmi le groupe constitué de l'azote, de l'oxygène et du soufre,
R61 représente un alkyl en C1-4 ou -T2-N(R611)R612, où
soit
T2 représente une liaison, et
R611 représente un hydrogène, alkyle en C1-4, hydroxy-alkyle en C2-4, alcoxy-C 1-4-alkyle-C2-4, phényle-alkyle en C1-4, ou Har1-alkyle en C1-4, où
Har1 est optionnellement substitué par R6111 et/ou R6112 et représente un cycle hétéroaromatique insaturé monocyclique ou bicyclique fusionné comportant 5 à 10 chaînons, comprenant un à trois hétéroatomes, chacun d'eux étant choisi parmi le groupe constitué de l'azote, de l'oxygène et du soufre, où
R6111 représente un halogène ou alkyle en C1-4,
R6112 représente un alkyle en C1-4, et
R612 représente un hydrogène, alkyle en C1-4, alcoxy-C1-4-alkyle-C2-4 ou hydroxy-alkyle en C2-4,
ou R611 et R612 ensemble et avec inclusion de l'atome d'azote, auquel ils sont liés, forment un cycle hétérocyclique Het1, où
Het1 est un groupe morpholino, thiomorpholino, S-oxo-thiomorpholino, S,S-dioxo- thiomorpholino, pipéridino, pyrrolidino, pipérazino ou 4N-(alkyle C1-4)-pipérazino,
soit
T2 représente un alkylène en C1-4 ou alkylène en C2-4 interrompu par un atome d'oxygène, et
R611 représente un hydrogène, alkyle en C1-4, hydroxy-alkyle en C2-4, alcoxy-C 1-4-alkyle-C2-4, phényle-alkyle en C1-4 ou Har1-alkyle en C1-4, où
Har1 est optionnellement substitué par R6111 et/ou R6112 et représente un cycle hétéroaromatique insaturé monocyclique ou bicyclique fusionné comportant 5 à 10 chaînons, comprenant un à trois hétéroatomes, chacun d'eux étant choisi parmi le groupe constitué de l'azote, de l'oxygène et du soufre, où
R6111 représente un halogène ou alkyle en C1-4,
R6112 représente un alkyle en C1-4, et
R612 représente un hydrogène, alkyle en C1-4, alcoxy-C1-4-alkyle-C2-4 ou hydroxy-alkyle en C2-4,
ou R611 et R612 ensemble et avec inclusion de l'atome d'azote, auquel ils sont liés, forment un cycle hétérocyclique Het1, où
Het1 représente un groupe morpholino, thiomorpholino, S-oxo-thiomorpholino, S,S-dioxo- thiomorpholino, pipéridino, pyrrolidino, pipérazino, 4N-(alkyle C1-4)-pipérazino, imidazolo, pyrrolo, triazolo ou pyrazolo,
R62 représente un alkyle en C1-4, alcoxy en C1-4, halogène, cyano, alcoxy-C 1-4-alkyle-C 1-4, (alkyle C1-4)-aminocarbonyle ou (alkyle C1-4)-aminosulfonyle,
R7 représente un hydroxyle ou Cyc1, où
Cyc1 représente une structure cyclique de formule Ia 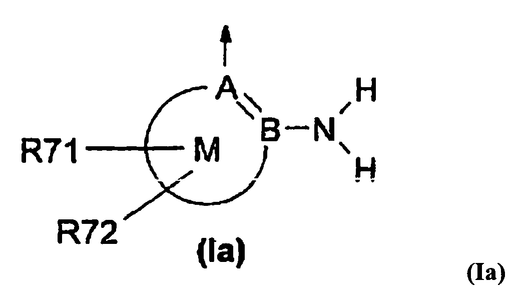
où
A représente C (carbone),
B représente C (carbone),
R71 représente un hydrogène, halogène, alkyle en C1-4 ou alcoxy en C1-4,
R72 représente un hydrogène, halogène, alkyle en C1-4 ou alcoxy en C1-4,
M avec inclusion de A et de B représente soit un cycle Ar2 soit un cycle Har2, où
Ar2 représente un cycle benzénique,
Har2 représente un cycle hétéroaromatique insaturé monocyclique à 5 ou 6 chaînons, comprenant un à trois hétéroatomes, chacun d'eux étant choisi parmi le groupe constitué d'azote, d'oxygène et de soufre,
et les sels de ces composés, dans lesquels l'halogène est choisi parmi un groupe constitué de brome, de chlore et de fluor.

2. Composés de la formule I conformément à la revendication 1,
où
R1 représente un hydrogène ou alkyle en C1-4,
R2 représente un hydrogène ou alkyle en C1-4,
R3 représente un hydrogène ou alkyle en C1-4,
R4 représente un hydrogène ou alkyle en C1-4,
R5 représente un hydrogène ou alkyle en C1-4,
R6 représente -T1-Q1, où
T1 représente une liaison,
Q1 représente un naphtyle, HAR, AR substitué par R61, AR substitué par R62 ou AR substitué par R61 et R62, où
AR représente un naphtyle ou HAR, où
HAR représente soit
un cycle hétéroaromatique insaturé monocyclique à 5 chaînons comprenant un, deux ou trois hétéroatomes, chacun d'eux étant choisi parmi un groupe constitué d'azote, d'oxygène et de soufre, ou
un cycle hétéroaromatique insaturé monocyclique à 6 chaînons comprenant un ou deux atomes d'azote, ou
un cycle hétéroaromatique insaturé bicyclique fusionné à 9 chaînons comprenant un, deux ou trois hétéroatomes, chacun d'eux étant choisi parmi un groupe constitué d'azote, d'oxygène et de soufre, ou
un cycle hétéroaromatique insaturé bicyclique fusionné à 10 chaînons comprenant un ou deux hétéroatomes, chacun d'eux étant choisi parmi un groupe constitué d'azote, d'oxygène et de soufre,
R61 représente un alkyle en C1-4, ou -T2-N(R611) R612, où
T2 représente une liaison ou un alkylène en C1-4,
R611 représente un hydrogène, alkyle en C1-4, phényle-alkyle en C1-4 ou Har1-alkyle en C1-4, où
Har1 représente soit
un cycle hétéroaromatique insaturé monocyclique à 5 chaînons comprenant un, deux ou trois hétéroatomes, chacun d'eux étant choisi parmi un groupe constitué d'azote, d'oxygène et de soufre, ou
un cycle hétéroaromatique insaturé monocyclique à 6 chaînons comprenant un ou deux atomes d'azote, ou
un cycle hétéroaromatique insaturé bicyclique fusionné à 9 chaînons comprenant un, deux ou trois hétéroatomes, chacun d'eux étant choisi parmi un groupe constitué d'azote, d'oxygène et de soufre, ou
un cycle hétéroaromatique insaturé bicyclique fusionné à 10 chaînons comprenant un ou deux hétéroatomes, chacun d'eux étant choisi parmi un groupe constitué d'azote, d'oxygène et de soufre,
R612 représente un hydrogène, alkyle en C1-4 ou hydroxy-alkyle en C2-4,
ou R611 et R612 ensemble et avec inclusion de l'atome d'azote, auquel ils sont liés, forment un cycle hétérocyclique Het1, où
Het1 représente un groupe morpholino, pipéridino, pyrrolidino, pipérazino ou 4N-méthyle- pipérazino,
R62 représente un alkyle en C1-4, alcoxy en C1-4 ou halogène,
R7 représente un hydroxyle ou 2-aminophényle,
et les sels de ces composés.

3. Composés de la formule I conformément à la revendication 1,
où
R1 représente un hydrogène,
R2 représente un hydrogène,
R3 représente un hydrogène,
R4 représente un hydrogène,
R5 représente un hydrogène,
R6 représente -T1-Q1, où
T1 représente une liaison,
Q1 représente un naphtyle, HAR, AR substitué par R61, N-méthyle-imidazolyle, N-méthyle- pyrazolyle, N-méthyle-indolyle, thiazolyle substitué par mono- ou di-méthyle, N-méthyle- imidazolyle substitué par méthyle ou N-méthyle-pyrazolyle substitué par méthyle, où
AR représente un naphtyle ou HAR, où
HAR représente un groupe pyridinyle, thiazolyle, benzothiophényle, benzothiazolyle, benzofuranyle, indolyle, quinolinyle ou isoquinolinyle,
R61 est un alkyle en C1-4 ou -T2-N(R611)R612, où
T2 représente une liaison ou un alkylène en C1-2,
R611 représente un hydrogène ou alkyle en C1-4,
R612 représente un hydrogène ou alkyle en C1-4,
ou R611 et R612 ensemble et avec inclusion de l'atome d'azote, auquel ils sont liés, forment un cycle hétérocyclique Het1, où
Het1 représente un groupe morpholino, pipéridino, pyrrolidino, pipérazino ou 4N-méthyle- pipérazino,
R7 représente un hydroxyle ou 2-aminophényle,
et les sels de ces composés.

4. Composés de la formule I conformément à la revendication 1,
où
R1 représente un hydrogène,
R2 représente un hydrogène,
R3 représente un hydrogène,
R4 représente un hydrogène,
R5 représente un hydrogène,
R6 représente -T1-Q1, où
T1 représente une liaison,
Q1 représente un naphtyle, HAR, pyridinyle substitué par R61, N-méthyle-imidazolyle, N- méthyle-pyrazolyle, thiazolyle substitué par mono- ou di-méthyle, N-méthyle-imidazolyle substitué par méthyle ou N-méthyle-pyrazolyle substitué par méthyle, où
HAR représente un groupe pyridinyle, thiazolyle, benzothiophényle ou benzothiazolyle,
R61 représente -T2-N(R611)R612, où
T2 représente une liaison ou un alkylène en C1-2,
R611 représente un hydrogène ou alkyle en C1-2,
R612 représente un hydrogène ou alkyle en C1-2,
ou R611 et R612 ensemble et avec inclusion de l'atome d'azote, auquel ils sont liés, forment un cycle hétérocyclique Het1, où
Het1 représente un groupe morpholino, pipéridino, pyrrolidino, pipérazino ou 4N-méthyle- pipérazino,
R7 représente un hydroxyle ou 2-aminophényle,
et les sels de ces composés.

5. Composés de la formule I conformément à la revendication 1,
où
R1 représente un hydrogène,
R2 représente un hydrogène,
R3 représente un hydrogène,
R4 représente un hydrogène,
R5 représente un hydrogène,
R6 représente -T1-Q1, où
T1 représente une liaison,
Q1 représente un naphtyle, HAR, 2-(R61)-pyridine-3-yle, 1-méthyle-pyrazole-4-yle, 1-méthyle- imidazole-4-yle, 1,2-di-méthyle-imidazole-4-yle ou 2,4-di-méthyle-thiazole-5-yle, où
HAR représente un groupe pyridine-3-yle, benzothiophène-2-yle ou benzothiazole-6-yle,
R61 représente -T2-N(R611)R612, où
T2 représente une liaison ou un méthylène,
R611 représente un hydrogène ou méthyle,
R612 représente un hydrogène ou méthyle,
ou R611 et R612 ensemble et avec inclusion de l'atome d'azote, auquel ils sont liés, forment un cycle hétérocyclique Het1, où
Het1 représente un groupe morpholino,
R7 représente un hydroxyle ou 2-aminophényle,
et les sels de ces composés.

6. Composés de la formule I conformément à la revendication 1,
où
R1 représente un hydrogène,
R2 représente un hydrogène,
R3 représente un hydrogène,
R4 représente un hydrogène,
R5 représente un hydrogène,
R6 représente -T1-Q1, où
T1 représente une liaison,
Q1 représente un naphtyle, HAR ou 2-(R61)-pyridine-3-yle, où
HAR représente un pyridinyle,
R61 représente -T2-N(R611) R612, où
T2 représente une liaison ou un méthylène,
R611 représente un hydrogène ou méthyle,
R612 représente un hydrogène ou méthyle,
ou R611 et R612 ensemble et avec inclusion de l'atome d'azote, auquel ils sont liés, forment un cycle hétérocyclique Het1, où
Het1 représente un groupe morpholino,
R7 représente un hydroxyle ou 2-aminophényle,
et les sels de ces composés.

7. Un composé de la formule I conformément à la revendication 1 qui est choisi parmi les
suivants :
(E)-N-hydroxy-3-[1-(naphtalène-2-sulfonyle)-1H-pyrrole-3-yle]-acrylamide,
(E)-N(2-aminophényle)-3-[1-(naphtaléne-2-sulfonyle)-1H-pyrrole-3-yle]-acrylamide,
(E)-N-hydroxy-3-[1-(pyridine-3-sulfonyle)-1H-pyrrole-3-yle]-acrylamide,
(E)-N-hydroxy-3-[1-(6-morpholine-4-yle-pyridine-3-sulfonyle)-1H-pyrrole-3-yle]-acrylamide,
(E)-N-(2-aminophényle)-3-[1-(benzo[b]thiophène-2-sulfonyle)-1H-pyrrole-3-yle]-acrylamide,
(E)-N-(2-aminophényle)-3-[1-(benzothiazole-6-sulfonyle)-1H-pyrrole-3-yle]-acrylamide,
(E)-N-hydroxy-3-[1-(1-méthyle-1H-imidazole-4-sulfonyle)-1H-pyrrole-3-yle]-acrylamide,
(E)-N-hydroxy-3-[1-(1-méthyle-1H-pyrazole-4-sulfonyle)-1H-pyrrole-3-yle]-acrylamide,
(E)-3-[1-(benzo[b]thiophène-2-sulfonyle)-1H-pyrrole-3-yle]-N-hydroxy-acrylamide,
(E)-3-[1-(benzothiazole-6-sulfonyle)-1H-pyrrole-3-yle]-N-hydroxy-acrylamide,
(E)-3-[1-[2,4-di-méthyle-thiazole-5-sulfonyle)-1H-pyrrole-3-yle]-N-hydroxy-acrylamide, et
(E)-3-[1-[1,2-di-méthyle-1H-imidazole-4-sulfonyle)-1H-pyrrole-3-yle]-N-hydroxy-acrylamide,
et un de ses sel.

8. Composés tels que revendiqués dans l'une quelconque des revendications 1 à 7, destinés à être utilisés dans le traitement de maladies.

9. Une composition pharmaceutique comprenant un ou plusieurs composés tels que revendiqués dans l'une quelconque des revendications 1 à 7 conjointement avec des excipients et/ou agents secondaires usuels.

10. Utilisation de composés tels que revendiqués dans l'une quelconque des revendications 1 à 7 pour la fabrication de compositions pharmaceutiques destinées au traitement de maladies réagissant à l'inhibition de l'activité histone déacétylase ou sensibles à ladite inhibition.

11. Utilisation de composés tels que revendiqués dans l'une quelconque des revendications 1 à 7 pour la fabrication de compositions pharmaceutiques destinées au traitement de néoplasies bénignes et/ou malignes, comme par ex. le cancer.

12. Un composé tel que revendiqué dans l'une quelconque des revendications 1 à 7 destiné au traitement, à la prévention ou à l'amélioration de maladies hyperprolifératives de type bénin ou malin et/ou de troubles réagissant au déclenchement d'une apoptose, comme par ex. une néoplasie bénigne ou maligne, par ex. le cancer, chez un patient.

13. Pour le traitement de néoplasies bénignes et/ou malignes - comme par ex. le cancer - chez un patient, à l'occasion duquel une quantité thérapeutiquement efficace et tolérable dudit composé doit être administrée audit patient optionnellement, simultanément, consécutivement ou séparément à un ou plusieurs autres agents thérapeutiques.

14. Une combinaison comprenant un premier ingrédient actif étant au moins un composé conformément à l'une quelconque des revendications 1 à 7, et un deuxième ingrédient actif étant au moins un agent anticancéreux choisi parmi le groupe constitué d'agents anticancéreux chimiothérapeutiques et d'agents anticancéreux spécifiques à une cible, pour une utilisation thérapeutique séparée, consécutive, simultanée, concomitante ou chronologiquement échelonnée, comme par ex. dans une thérapie contre une néoplasie bénigne ou maligne, par ex. le cancer.

15. Pour le traitement, la prévention ou l'amélioration de maladies hyperprolifératives et/ou de troubles réagissant au déclenchement d'une apoptose, comme par ex. une néoplasie bénigne ou maligne - par ex. le cancer - chez un patient, à l'occasion de quoi ledit patient en ayant besoin reçoit une administration séparée, simultanée, concomitante, consécutive ou chronologiquement échelonnée d'une certaine quantité d'un premier composé actif correspondant à un composé d'après l'une quelconque des revendications 1 à 7, et d'une certaine quantité d'au moins un deuxième composé actif, ledit deuxième composé actif étant un agent anticancéreux choisi parmi un groupe constitué d'agents anticancéreux chimiothérapeutiques et d'agents anticancéreux spécifiques à une cible ; à cette occasion, les quantités respectives du premier composé actif et dudit deuxième composé actif doivent permettre d'obtenir un effet thérapeutique.

16. La combinaison ou un composé conformément aux revendications 14 ou 15 pour le traitement, la prévention ou l'amélioration de maladies hyperprolifératives et/ou de troubles réagissant au déclenchement d'une apoptose, où lesdits agents anticancéreux chimiothérapeutiques sont choisis parmi (i) des agents alkylants/carbamylants incluant le cyclophosphamide,
l'ifosfamide, le thiotépa, le melphalan et la chloroéthylnitrosourée ; (ii) les dérivés du platine incluant le cisplatine, l'oxaliplatine, et le carboplatine ; (iii) les agents antimitotiques / inhibiteurs de tubuline incluant les alcaloïdes de la pervenche, comme par ex. la vincristine, la vinblastine ou la vinorelbine, les taxanes, comme par ex. le paclitaxel, le docétaxel et analogues de même que les formulations et conjugués de ceux-ci, les épothilones, comme par ex. l'épothilone B, l'azaépothilone ou le ZK-EPO ; (iv) les inhibiteurs de topoisomérase incluant les anthracyclines, comme par ex. la doxorubicine, les épipodophyllotoxines, comme par ex. l'étoposide, la camptothécine et les analogues de la camptothécine, comme par ex. l'irinotécan ou le topotécan ; (v) les antagonistes de la pyrimidine incluant le 5-fluorouracile, la capécitabine, l'arabinosylcytosine / cytarabine et la gemcitabine ; (vi) les antagonistes de la purine incluant la 6-mercaptopurine, la 6-thioguanine et la fludarabine ; et (vii) les antagonistes de l'acide folique incluant le méthotrexate et le permetrexed.

17. La combinaison ou un composé selon les revendications 14, 15 ou 16 pour le traitement, la prévention ou l'amélioration de maladies hyperprolifératives et/ou de troubles réagissant au déclenchement d'une apoptose, où lesdits agents anticancéreux spécifiques à une cible sont choisis parmi (i) les inhibiteurs de kinase, incluant l'imatinib, ZD-1839 / géfitinib, BAY43-9006 / sorafénib, Su11248 / sunitinib et OSI-774 / erlotinib ; (ii) les inhibiteurs du protéasome incluant PS-341 / bortézomib ; (iii) les inhibiteurs d'histone déacétylase incluant SAHA, PXD101, MS275, MGCD0103, depsipeptide / FK228, NVP-LBH589, NVP-LAQ824, l'acide valproïque (VPA) et les butyrates ; (iv) les inhibiteurs de la protéine de choc thermique 90 incluant la 17-allylaminogeldanamycine (17-AAG) ; (v) les agents de ciblage vasculaire (VTA) incluant la combrétastatine A4 phosphate et AVE8062 / AC7700, et les médicaments anti-angiogéniques incluant les anticorps VEGF, comme par ex. le bévacizumab, et les inhibiteurs de tyrosine kinase KDR, comme par ex. PTK787 / ZK222584 (vatalanib) ; (vi) les anticorps monoclonaux incluant le trastuzumab, le rituximab, l'alemtuzumab, le tositumab, le cétuximab et le bévacizumab de même que les mutants et conjugués d'anticorps monoclonaux, comme par ex. le gemtuzumab ozogamicine ou l'ibritumomab tiuxétan, et les fragments d'anticorps ; (vii) les thérapeutiques à base d'oligonucléotides incluant G-3139 / oblimersen ; (viii) les agonistes de récepteurs Toll-like / TLR 9 incluant le Promue®, les agonistes de TLR 7 incluant l'imiquimod et l'isatoribine et leurs analogues, ou les agonistes de TLR 7/8 parmi lesquels le résiquimod de même que l'ARN immunostimulant en tant qu'agoniste de TLR 7/8 ; (ix) les inhibiteurs de protéase ; (x) les thérapeutiques hormonales incluant les anti-oestrogènes, comme par ex. le tamoxifène ou le raloxifène, les anti-androgènes, comme par ex. le flutamide ou le casodex, les analogues de LHRH, comme par ex. le luprolide, la goséréline ou la triptoréline, et les inhibiteurs d'aromatases ;
la bléomycine ; les rétinoïdes incluant l'acide tout-trans-rétinoïque (ATRA) ; les inhibiteurs d'ADN méthyltransférase incluant le dérivé de 2-désoxycytidine décitabine et la 5-azacytidine ; l'alanosine ; les cytokines, incluant l'interleukine 2 ; les interférons incluant l' interféron α2 et l'interféron y ; et les agonistes de récepteurs de mort incluant TRAIL, les anticorps agonistiques de DR4/5, FasL et les agonistes TNF-R.

18. L'utilisation, un composé ou une combinaison conformément à l'une quelconque des revendications 11 à 15 pour le traitement, la prévention ou l'amélioration de maladies hyperprolifératives et/ou de troubles réagissant au déclenchement d'une apoptose, où ledit cancer fait partie du groupe constitué du
cancer du sein, de la vessie, des os, du cerveau, du système nerveux central et périphérique, du côlon, des glandes endocrines, de l'oesophage, de l'endomètre, des cellules germinales, de la tête et du cou, du rein, du foie, du poumon, du larynx et de l'hypopharynx, du mésothéliome, du sarcome, de l'ovaire, du pancréas, de la prostate, du rectum, rénal, de l'intestin grêle, des tissus mous, du testicule, de l'estomac, de la peau, de l'urètre, du vagin et de la vulve ;
des cancers héréditaires, du rétinoblastome et de la tumeur de Wilms ;
de la leucémie, du lymphome, des lymphomes non hodgkiniens, de la leucémie myéloïde chronique et
aiguë, de la leucémie lymphoblastique aiguë, de la maladie de Hodgkin, du myélome multiple et du lymphome à lymphocytes T ;
du syndrome myélodysplasique, de la néoplasie à plasmocytes, des syndromes paranéoplasiques, des cancers de site primaire inconnu et des tumeurs malignes liées au SIDA.

19. Utilisation de composés tels que revendiqués dans l'une quelconque des revendications 1 à 7 pour la fabrication de compositions pharmaceutiques destinées au traitement de maladies autres que la néoplasie maligne, comme par ex. les arthropathies et maladies ostéopathologiques, les maladies auto-immunes incluant les rejets de transplantations, les maladies inflammatoires chroniques et aiguës, les maladies hyperprolifératives ou les troubles neuropathologiques.
